# EUROPÄISCHE PATENTSCHRIFT

(11) **EP 4 401 653 B1**
(45) Veröffentlichungstag und Bekanntmachung des Hinweises auf die Patenterteilung: **03.09.2025**
(21) Anmeldenummer: 22786791.8
(22) Anmeldetag: 16.09.2022
(51) Int. Cl.: A61B 17/16

(54) **MEDIZINISCHES HANDINSTRUMENT**
HAND-HELD MEDICAL INSTRUMENT
INSTRUMENT MÉDICAL À MAIN

(30) Priorität: 19.09.2021 DE 202021003055 U; 10.01.2022 DE 202022100107 U; 26.01.2022 DE 202022100457 U
(43) Veröffentlichungstag der Anmeldung: 24.07.2024
(73) Patentinhaber: El-Khatib, Walid, 12107 Berlin (DE)
(72) Erfinder: El-Khatib, Walid, 12107 Berlin (DE)
(74) Vertreter: Eisenführ Speiser
(86) Internationale Anmeldenummer: PCT/EP2022/075839
(87) Internationale Veröffentlichungsnummer: WO 2023/041741

(56) Entgegenhaltungen:
- EP-A1- 1 829 486
- WO-A1-97/11646
- DE-U1- 8 531 992

## Beschreibung

Die Erfindung betrifft ein medizinisches Handinstrument, insbesondere einen Einwegknochenhobel, mit ein einem Handstück und einer distal des Handstücks angeordneten Hobelklinge und Spänekammer.

Derartige Handinstrumente werden beispielsweise als Knochenschaber in der Zahnmedizin eingesetzt, um Knochenmaterial für eine Eigenknochentransplantation zu gewinnen. Beispielsweise wird die Transplantation von Eigenknochen genutzt, um eine stabile Knochenstruktur für Zahnimplantate zu schaffen.

Die Hobelklinge des Handinstruments ist zum Schaben insbesondere von Knochen ausgebildet und so angeordnet, dass mittels der Hobelklinge geschabte Späne in die Spänekammer gelangen und dort für die weitere Verwendung gesammelt werden. Bei bekannten Handinstrumenten verläuft die Hobelklinge in einer gegenüber einer Längsachse des Handstücks geneigten Ebene. Ein derartiges Handinstrument ist beispielsweise aus EP 1 829 486 A1 bekannt.

Die Verwendung eines derartigen Handinstruments verlangt vom Nutzer, z.B. einem Zahnarzt oder Kieferchirurgen, eine große Ausdauer und Präzision in der Handhabung.

Der Erfinder hat erkannt, dass bekannte medizinische Handinstrumente diesbezüglich defizitär sind und dem Nutzer die Arbeit nicht im gewünschten Maße erleichtern.

Daher ist es Ziel der Erfindung, ein medizinisches Handinstrument zu schaffen, das den Nutzer entlastet.

Erfindungsgemäß wird dieses Ziel durch ein Handinstrument der eingangs genannten Art erreicht, bei dem das Handstück als Griff mit drei Fingerauflagen und einer proximalen Stützauflage ausgebildet ist, wobei die drei Fingerauflagen so zueinander geneigt sind, dass die drei Fingerauflagen in einem Querschnitt durch das Handstück paarweise in einem Winkel von kleiner als 90° zueinander verlaufen.

Die Erfindung schließt die Erkenntnis ein, dass auf ein dem zahnmedizinischen Markt aktuell vorhandenes Instrument zur Gewinnung von Knochenspäne aus dem Kiefer folgende Defizite aufweist:
Das Handstück ist ein gerader Stab. In der Handhabung führt dies beim Gebrauch zur Ermüdung der Finger des Zahnarztes. Der Erfinder hat erkannt, dass eine adäquate Form und Griffigkeit für die Finger und die Handführung fehlen, um effizient feinmotorische Bewegungen beim Abtrag zu vollziehen. Der Abtrag ist eher ein Kraftakt. - Durch die fehlende Ergonomie ist die Effektivität des Instruments in Abtragwinkel, Haltungswinkel und Führungswinkel erschwert. Das umliegende Gewebe kann und wird öfter verletzt.

Das Instrument beruht auf dem Prinzip des Schabens, was für den Abtrag eine größere Kraft bei geringerem Ertrag erfordert. Der Schabwinkel liegt hier bei 160°. Der Schnittwinkel der Schneide ist nicht optimal und zwingt den Zahnarzt, das Instrument in einem einzigen definierten Winkel zu benutzen. Dies ist aber anatomisch manchmal schwierig und führt auch zur Verletzung von Weichgewebe beim Schaben.

Die Hobelklinge ist nicht aus federhartem Stahl und in der Schärfe weit nicht ausgereizt. Außerdem ist sie schnell stumpf.

Das Instrument ist nur für Rechtshänder konzipiert.

Die Spänekammer zur Aufnahme der Späne kann nur ein Volumen von 2,5 - 3 cm³ aufnehmen.

Die Arbeitsfläche ist zu groß, was einen größeren chirurgischen Schnitt erfordert, um in das Spendergebiet arbeiten zu können.

Zur Lösung vorgenannter Probleme wurde der ergonomische Einwegknochenhobel aus der praktischen Erfahrung des Erfinders entwickelt. Aspekte dieses medizinischen Handinstruments sind die Folgenden:
Die Form des Instruments richtet sich nach ergonomischen Prinzipien. Mit dem Instrument sind durch die Form feinmotorische Bewegungen der Finger des Nutzers möglich. Das Instrument liegt durch die ergonomische Form gut in der Hand und kann deshalb auch feinmotorisch geführt werden.

Vorzugsweise sind die Fingerauflagen als wenigstens in Längsrichtung des Instruments gewölbte Mulden ausgebildet. Durch Mulden für die Finger und vorzugsweise zusätzlich Noppen für die Griffigkeit wird die benötigte Stabilität und der Halt in der Hand erreicht. Die Mulde an der Unterseite ist für die Auflage in den Bereich zwischen Daumen und Zeigefinger. Sowohl Rechts- als auch Linkshänder haben eine kontrollierte Führung beim Arbeiten. Dies führt zu wesentlich weniger Traumata des Weichgewebe.

Ein bevorzugter Abtragswinkel der Hobelklinge von 50° bis 75°, entsteht aus einer gedachten gezogenen Linie durch das Handstück in Bezug zur Schneide mit 18° bis 25°. Die Hobelklinge liegt im ca. 40° zum übrigen Handstück geneigten Aufnahmebereich von Hobelklinge und Späneaufnahmespalt. Dies erlaubt eine bessere feinmotorische Kontrolle. Diese Winkel können aber auch durch die Ergonomie leicht der anatomischen Situation durch Änderung der Fingerstellung in den Mulden angepasst werden. Durch das Prinzip des Hobelns ist ein qualitativ und quantitativ besserer Abtrag möglich. Der Abtragswinkel entspricht dem wie beim Hobeln und liegt bei 50° bis 75°. Dadurch ist auch wesentlich weniger Kraft notwendig, was die Finger des Chirurgen entlastet und mehr Feinmotorik zulässt.

Die Hobelklinge ist aus federhartem Stahl und merklich schärfer. Diese Hobelklinge verliert beim Arbeiten kaum an Schärfe.

Die Spänekammer kann in einer bevorzugten Ausführungsform ein Volumen von mehr als 3,5 cm³, vorzugsweise mehr als 4 cm³ aufweisen. Wenn die Spänekammer beispielsweise ein Volumen von 4 cm³ aufnehmen kann, bedeutet dies gegenüber dem Stand der Technik auch weniger Arbeitsunterbrechung, um die Spänekammer zu leeren.

Die Arbeitsfläche an der Spitze des Instruments ist schmal - vorzugsweise 0,5 mm bis 0,8 mm, z.B. 0,7 mm - und nur 16 mm bis 20 mm, beispielsweise 18,9 mm lang. Dies führt zu mehr Sicht beim Abtrag, dadurch mehr Kontrolle, Führung und keine Verletzung von Weichgewebe. Vorteilhaft ist auch, dass der Chirurg ein geringeres Operationsgebiet braucht, bedeutet weniger Schneiden, weniger Schwellung und weniger Schmerzen. Die angehängten Figuren und Erläuterungen stellen das Beschriebene nochmals dar.

Vorzugsweise sind die drei Fingerauflagen als Mulden ausgebildet und haben in Längsrichtung des Handstücks eine konkave Form.

Die Stützauflage ist vorzugsweise von einer balligen Fläche am proximalen Ende des Handstücks gebildet, die in diejenige radiale Richtung des Handstücks orientiert ist, in die eine äußere Flachseite der Hobelklinge orientiert ist. Besonders bevorzugt ist es, wenn die Stützauflage in Längsrichtung des Handinstruments in einem stumpfen Winkel zwischen 0° und 10° in Bezug auf die äußere Flachseite der Hobelklinge verläuft.

Vorzugsweise beträgt die Breite des Handstücks im Bereich der Fingerauflagen zwischen 11 mm und 16 mm. Ebenfalls ist es bevorzugt, wenn die Breite des Handstücks im Bereich der Stützauflage zwischen 20 mm und 25 mm beträgt und in distale Richtung zunächst abnimmt und im Bereich der Fingerauflagen wieder zunimmt.

Die Hobelklinge verläuft vorzugsweise in Wesentlichem, insbesondere mit Ausnahme der Schneide, in einer Klingenebene, die in Bezug auf die Längsachse des Handstücks um mindestens 50° geneigt ist.

Das Handstück ist vorzugsweise aus thermoplastischem Kunststoff gefertigt, und zwar vorzugsweise aus in der Medizin zugelassenen Polycarbonat oder Polyamid, beispielsweise dem Polycarbonat Makrolon 2458 oder dem Polyamid ixef 1022.

Vorzugsweise sind die Spänekammer und das Handstück einstückig ausgebildet und Bestandteil eines einstückigen Grundkörpers, an dem die Hobelklinge und ein Spänekammerverschluss in Form eines abnehmbaren Deckels befestigt sind.

Gemäß einer Ausführungsvariante des Handinstruments ist die Hobelklinge als konusförmige Scheibe ausgebildet und an einem Schaft befestigt, der in dem Handstück längsverschieblich angeordnet ist. Die Umfangskante der konusförmige Scheibe bildet dabei die Schneidkante der Hobelklinge. Durch die Konusform der Hobelklinge ist auch der Schneidenwinkel der Hobelklinge definiert.

Bei dieser Ausführungsform ist die Spänekammer vorzugsweise als ein zylindrischer, den Schaft konzentrisch umgebender Hohlraum ausgebildet, der am distalen Ende des Handstücks offen ist. Mittels eines vorzugsweise vorgesehenen Auswerfers können Hobelspäne distal aus der Spänekammer ausgeworfen werden. Dazu ist der Auswerfer zusammen mit der Hobelklinge und dem Schaft längsverschieblich in dem Handstück angeordnet. Der Auswerfer hat einen axialen Abstand von der Hobelklinge und die Spänekammer erstreckt sich in axialer Richtung zwischen der Hobelklinge und einer Stirnwand des Auswerfers.

Vorzugsweise ist der Schaft mit der Hobelklinge derart arretierbar, dass der Schaft und die Hobelklinge (im arretierten Zustand nicht mehr bezüglich des Handstücks längsverschieblich sind und die Hobelklinge in einer zum Schaben dienenden Arbeitsposition derart arretiert ist, dass mittels des Handstücks Zugkräfte in den Schaft eingeleitet und auf die Hobelklinge übertragen werden können.

Der Schaft ist vorzugsweise wenigstens mittelbar mit einem Griff verbunden, der zum Längsverschieben des Schafts und der Hobelklinge bezüglich des Handstücks dient. Hierbei weist das Handstück vorzugsweise eine Längsnut zur Führung des Griffs auf. Außerdem weist das Handstück vorzugsweise eine Ausnehmung am proximalen Ende der Längsnut auf, in die der Griff mittels einer Drehbewegung um die Längsachse des Schafts herum hineingeschwenkt werden kann, um den Schaft und die Hobelklinge in der Arbeitsposition zu arretieren.

Bei einem Handinstrument mit einer Hobelklinge in Form einer konusförmigen Scheibe oder eines stumpfen Kegels ist das distale Ende des Handstücks, insbesondere des Grundkörpers des Handstücks stufenförmig gestaltet, wobei ein distal nach vorne abstehender Vorsprung des Grundkörpers einen ersten Teilumfang der Hobelklinge umschließt, während eine in Längsrichtung des Grundkörpers zurückspringende Stirnwand des Grundkörpers den Spalt, der zur Spänekammer führt, in Längsrichtung begrenzt. Der Spalt befindet sich somit zwischen der Schneidkante der Hobelklinge und der in axialer Richtung zurückversetzten Stirnwand des Grundkörpers des Handinstruments. Im Bereich des ersten Teilumfangs der Hobelklinge ist am distalen Ende des Grundkörpers außerdem vorzugsweise eine Stütz- und Anlagefläche für die Hobelklinge vorgesehen, die in einer senkrecht zur Längsachse des Schafts der Hobelklinge verlaufenden Ebene liegt, die gegenüber der Ebene, in der die den Spalt begrenzende Stirnfläche verläuft, um die Breite des Spalts in distale Richtung versetzt ist. Die Stütz- und Anlagefläche ist vorzugsweise von dem nach vorne abstehende Vorsprung des Grundkörpers eingefasst.

Beim Schaben wirken in dem Schaft der Hobelklinge Zugkräfte, die auf die Hobelklinge übertragen werden. Von dem abzuschaben Material werden der Zugkraft entgegen gerichtete Kräfte auf die Hobelklinge ausgeübt, die zu einem Drehmoment führen. Die Stütz- und Anlagefläche am distalen Ende des Grundkörpers fängt dieses Drehmoment auf und stützt die Hobelklinge. Der nach vorne abstehende Vorsprung des Grundkörpers fängt Kräfte auf, die quer zur Längsachse des Schafts auf die Hobelklinge einwirken. Durch den Schaft, die Stütz- und Anlagefläche und den nach vorne abstehende Vorsprung des Grundkörpers ist die Hobelklinge in ihrer Arbeitsposition für das Schaben optimal stabilisiert und so gehalten, dass die beim Schaben wirkenden Kräfte in den Grundkörper eingeleitet werden, ohne dass sich Schaft und Hobelklinge unter dem Einfluss der einwirkenden Kräfte stärker verformen können. Durch den den ersten Teilumfang der Schneidkante in der Arbeitsposition der Hobelklinge umfassenden Vorsprung am distalen Ende des Grundkörpers ist außerdem die Verletzungsgefahr gemindert.

Die ergonomische Form des Handstücks und die Ausgestaltung der Hobelklinge und des distalen Endes des Grundkörpers haben einen synergistischen Effekt und erlauben in Kombination miteinander eine besonders präzise und effektive Handhabung des Instruments, weil die Griffflächen an dem Grundkörper eine präzise Einleitung der Hand- und Fingerkräfte in das Instrument ermöglichen und der Aufbau der Hobelklinge in Kombination mit der Gestaltung des distalen Endes des Grundkörpers ein präzise Umsetzung der eingeleiteten Kräfte erlaubt.

Die Erfindung soll nun anhand eines Ausführungsbeispiels mit Bezug auf die Figuren näher erläutert werden. Von den Figuren zeigt:
- Fig. 1:: eine perspektivische Gesamtansicht des medizinischen Handinstruments;
- Fig. 2:: einen Längsschnitt durch das medizinische Handinstrument;
- Fig. 3:: eine Ansicht des medizinischen Handinstruments vorne und schräg oben;
- Fig. 4:: eine Aufsicht auf das medizinische Handinstrument;
- Fig. 5:: eine linke Seitenansicht des medizinischen Handinstruments;
- Fig. 6:: eine rechte Seitenansicht des medizinischen Handinstruments;
- Fig. 7:: eine Vorderansicht des medizinischen Handinstruments; und
- Fig. 8:: einen Querschnitt durch das medizinische Handinstrument im Bereich der Fingerauflagen
- Fig. 9:: Details der Instrumentenspitze in einer teilweise geschnittenen Seitenansicht,
- Fig. 10a - c:: verschiedene perspektivische Ansichten zur Illustration der Spänekammer des Handinstruments,
- Fig. 11:: einen Längsschnitt durch einen distalen Endabschnitt des medizinischen Handinstruments zur Illustration der Anordnung von Hobelklinge und Spänekammer;
- Fig. 12:: einen vergrößerten Ausschnitt des Längsschnitts aus Figur 11;
- Fig. 13:: ein Gittermodell eines distalen Längsabschnitts des Handinstruments zur Darstellung der räumlichen Anordnung der Hobelklinge und einer Befestigungsschraube für die Hobelklinge;
- Fig. 14:: eine alternative Ausführungsform eines im medizinischen Handgeräts ähnlich dem zuvor dargestellten jedoch mit einem größeren Winkel zwischen Klingenebene und Längsachse des Handstücks;
- Fig. 15:: eine weitere alternative Ausführungsform eines im medizinischen Handgeräts ähnlich dem zuvor dargestellten jedoch mit einem noch größeren Winkel zwischen Klingenebene und Längsachse des Handstücks:
- Fig. 16:: eine perspektivische Ansicht der Hobelklinge;
- Fig. 17:: einen schematischen Längsschnitt durch die Hobelklinge;
- Fig. 18:: verschiedene Ansichten der Befestigungsschraube zum Befestigen der Hobelklinge an dem Handinstrument;
- Fig. 19:: eine alternative Ausführungsform eines im medizinischen Handgeräts ähnlich dem zuvor dargestellten jedoch mit einem schmaler ausgeführten distalen Ende; und
- Fig. 20:: eine weitere alternative Ausführungsform des medizinischen Handinstruments mit einer Rundklinge, die an einer von dem übrigen Handinstrument abnehmbaren Spänekammer befestigt ist;
- Fig. 21:: eine perspektivische Gesamtansicht einer zweiten Variante des medizinischen Handinstruments schräg von oben;
- Fig. 22:: eine perspektivische Gesamtansicht der zweiten Variante des medizinischen Handinstruments schräg von der Seite;
- Fig. 23:: eine perspektivische Gesamtansicht der zweiten Variante des medizinischen Handinstruments schräg von unten;
- Fig. 24:: eine perspektivische Detailansicht des distalen Endes der zweiten Variante des medizinischen Handinstruments;
- Fig. 25:: ein Längsschnitt durch das distale Ende der zweiten Variante des medizinischen Handinstruments;
- Fig. 26:: Ansichten der vier Längsseiten des medizinischen Handinstruments a) von links, b) von oben, c) von rechts und d) von unten;
- Fig. 27:: eine perspektivische Explosionszeichnung des medizinischen Handinstruments;
- Fig. 28:: eine perspektivische Gesamtansicht der zweiten Variante des medizinischen Handinstruments schräg von vorn;
- Fig. 29:: eine perspektivische Gesamtansicht der zweiten Variante des medizinischen Handinstruments mit Hobelkling und Schaft in de rausgefahrenen Auswerf-Position; und
- Fig. 30:: eine alternative Gestaltung der Baueinheit aus Griff, Schaft, Auswerfer und Hobelklinge des medizinischen Handinstruments.

Ein erfindungsgemäßes medizinisches Handinstrument 10 besitzt einen vorzugsweise einstückig ausgebildeten Grundkörper 12, der sich von einem distalen Ende 14 des medizinischen Handinstruments 10 zu einem proximalen Ende 16 des medizinischen Handinstruments erstreckt. Nahe seinem distalen Ende 14 weist das medizinischer Handinstrument eine Hobelklinge 18 auf. Ein proximal der Hobelklinge 18 verlaufender Längsabschnitt des einstückigen Grundkörpers 12 ist als Handstück 20 ausgebildet. Das Handstück 20 dient zum Greifen des medizinischen Handinstruments 10 mit einer Hand.

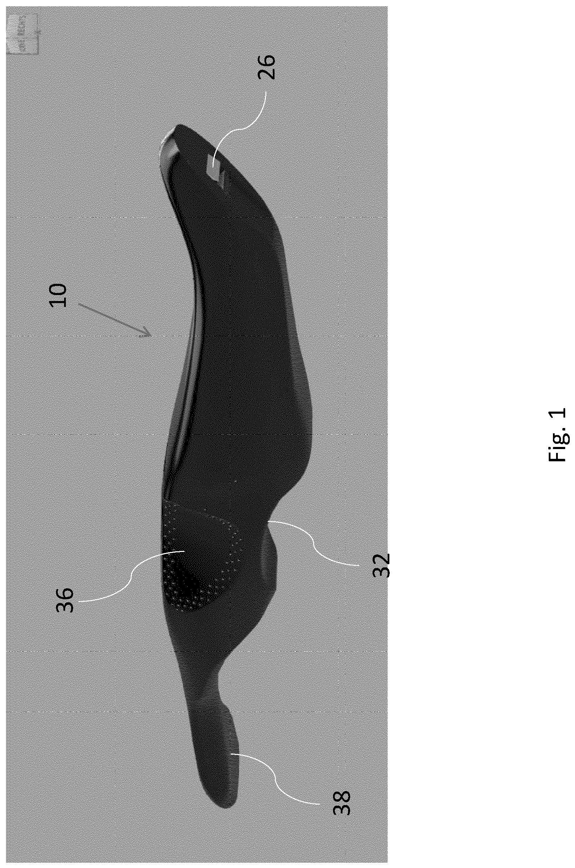

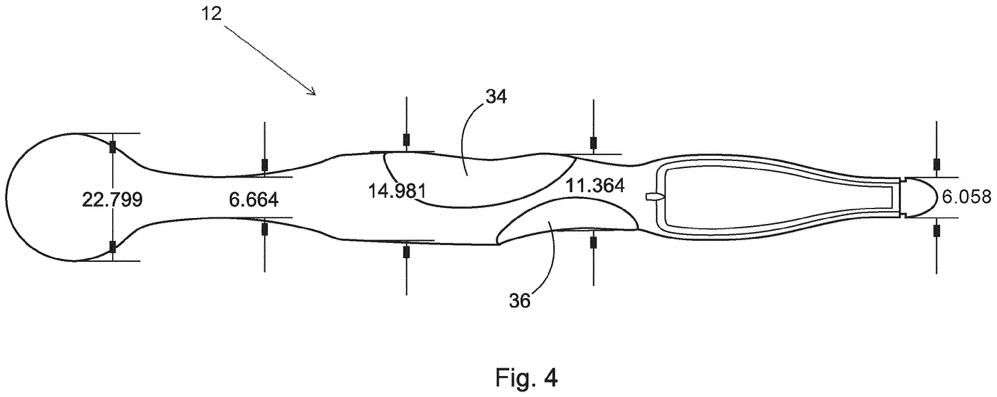

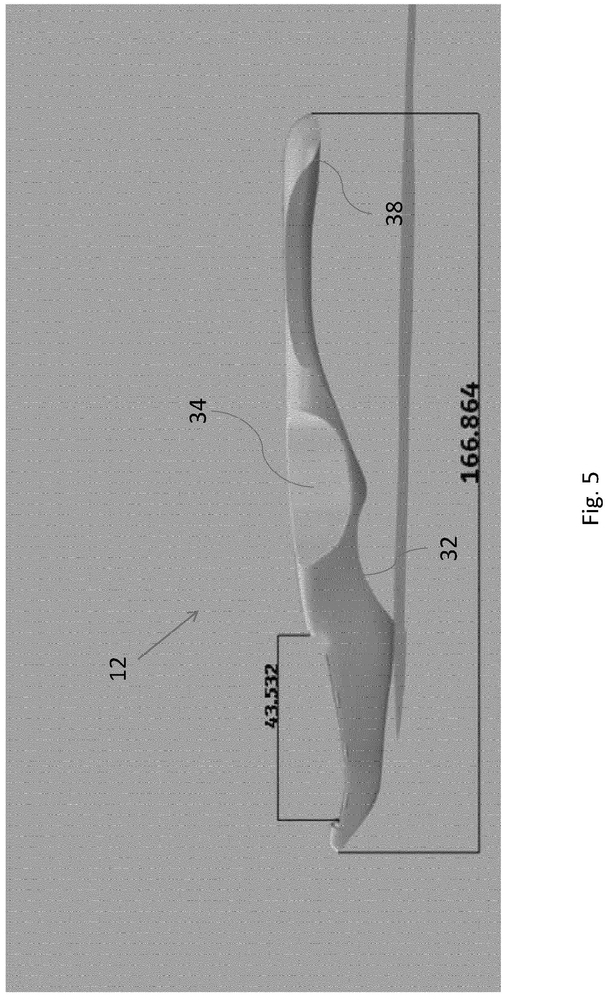

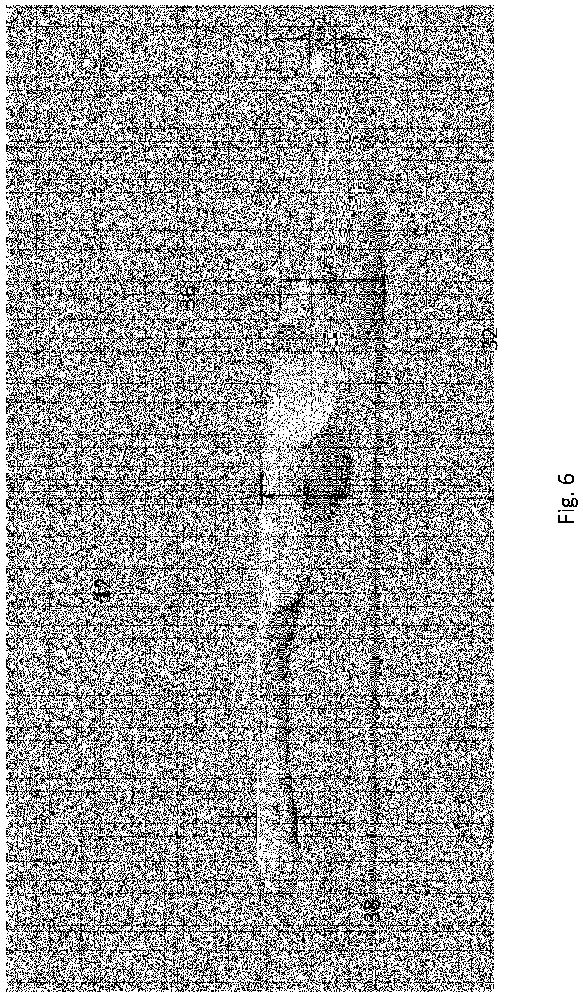

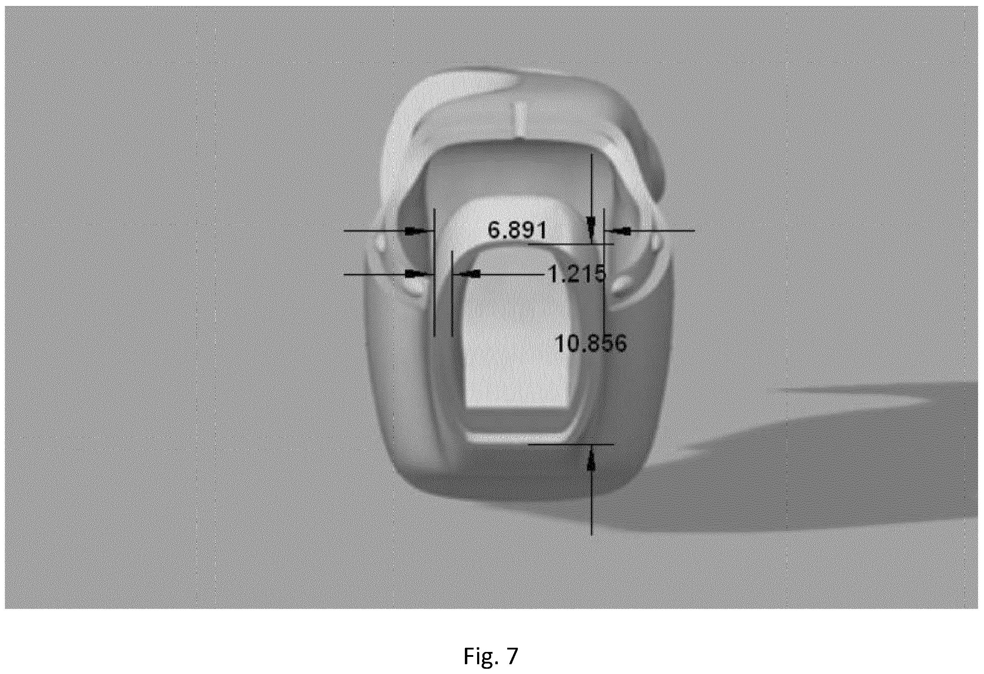

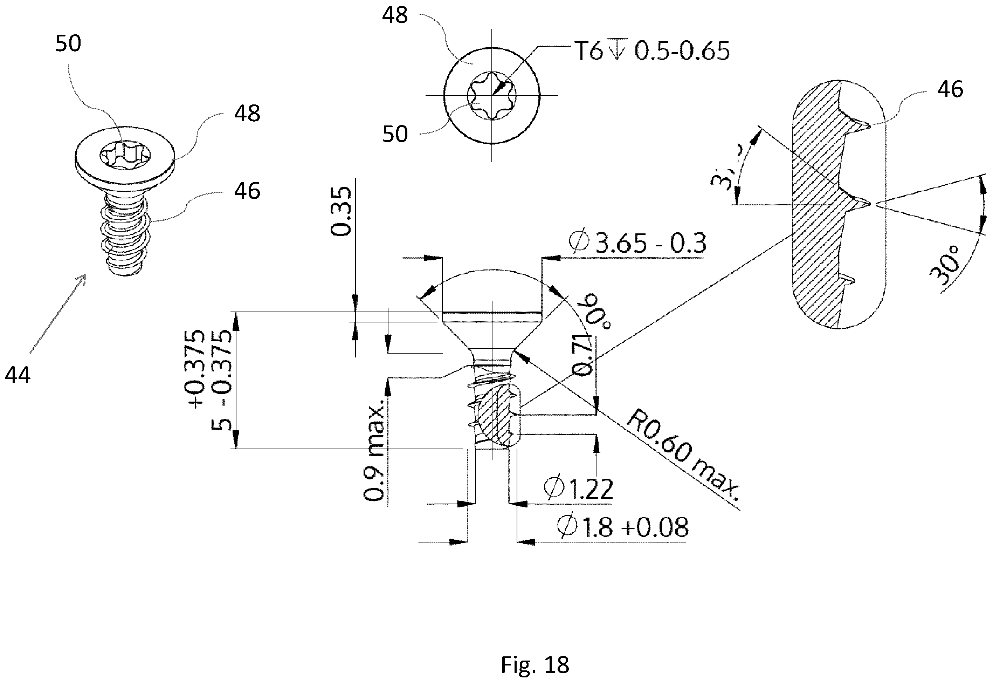

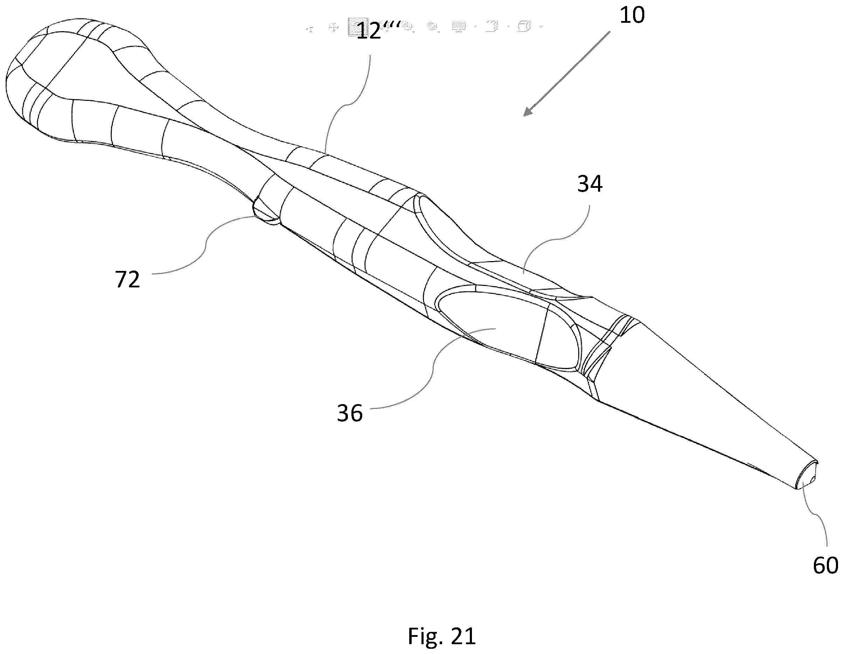

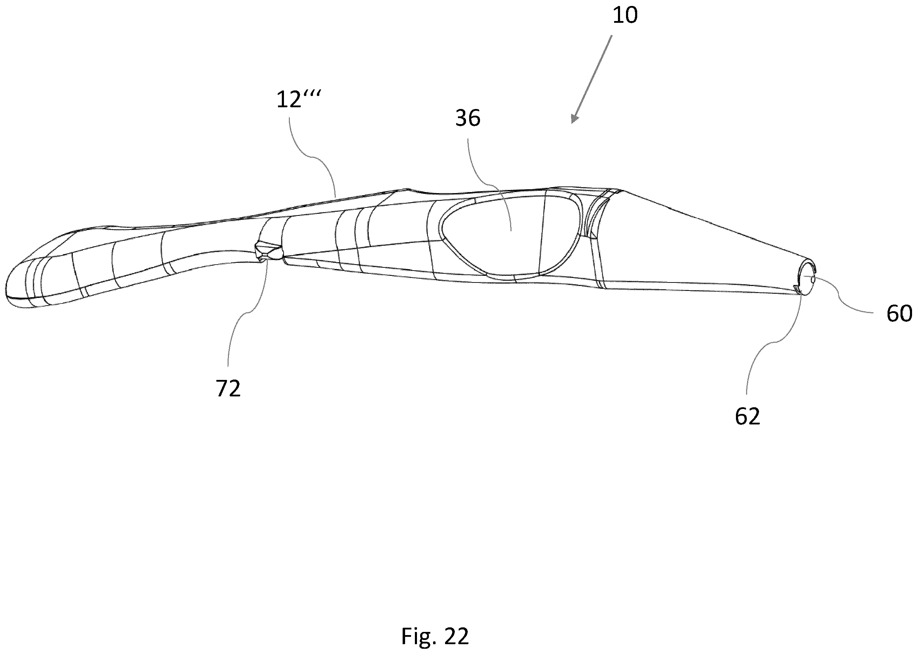

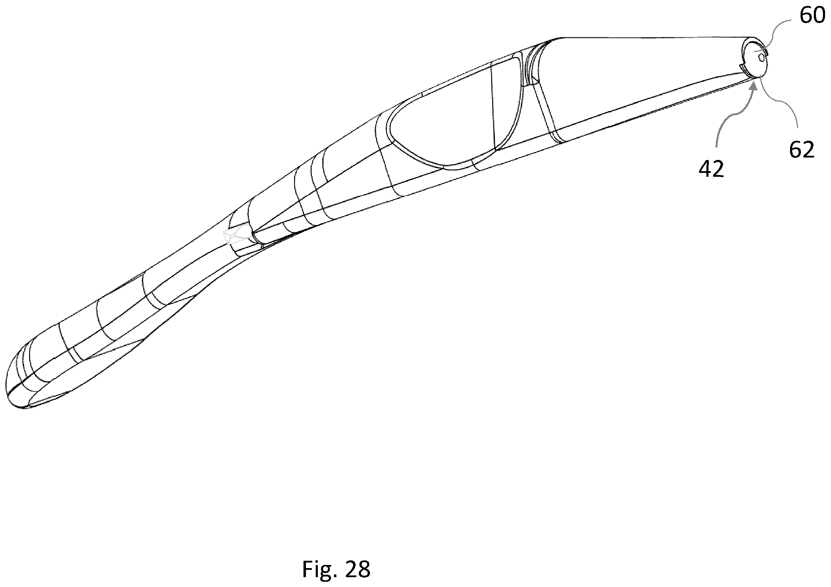

Vorgestellt werden zwei Ausführungsvarianten des medizinischen Handinstruments 10. Figuren 1 bis 20 zeigen eine erste Variante sowie Untervarianten der ersten Variante des medizinischen Handinstruments 10 und Details desselben, während Figuren 21 bis 29 eine zweite Variante des medizinischen Handinstruments 10 und Details desselben zeigen.

Die Unterschiede betreffen insbesondere die Hobelklinge, während der als Handstück 20 ausgebildete Längsabschnitt des einstückigen Grundkörpers 12 bei beiden Ausführungsvarianten zumindest ähnlich ist.

Zunächst wird die erste Variante des medizinischen Handinstruments 10 näher beschrieben:
Das Handstück 20 erstreckt sich entlang einer Längsachse 22 und die Hobelklinge 18 verläuft in einer Klingenebene 24. Aus der Klingenebene 24 ragt die Schneide 26 der Hobelklinge 18 heraus, sodass sich ein Schnittwinkel 28 zwischen der Schneide 26 und der Klingenebene 24 ergibt.

Vorzugsweise beträgt der Abtragswinkel der Hobelklinge, der zwischen 50° und 75° betragen soll, und der Führungswinkel beträgt vorzugsweise etwa 40°. Der Abtragswinkel α + β der Hobelklinge zwischen 50° und 75° ergibt sich aus der Lage der Schneide 26 mit dem Schnittwinkel α von 18° und einer horizontal gezogenen Linie durch das gesamte Handstück 20. Der Führungswinkel β, der etwa 40° betragen soll, entsteht aus der Neigung der Hobelklinge und Spalte zur Aufnahme der Späne umgebenden Bereiches (entsprechend der Klingeneben 24) in Bezug zum Handstück 20. Der Führungswinkel β ist somit die Neigung der Instrumentenspitze zum übrigen Instrument 10.

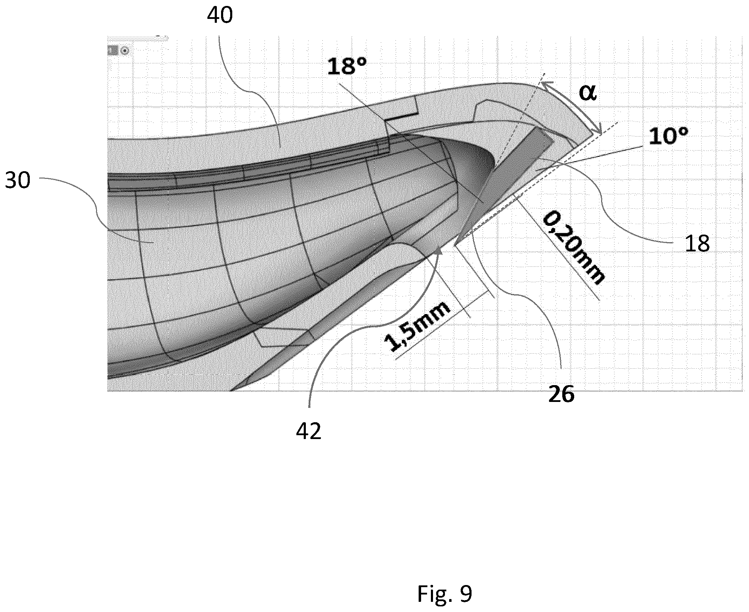

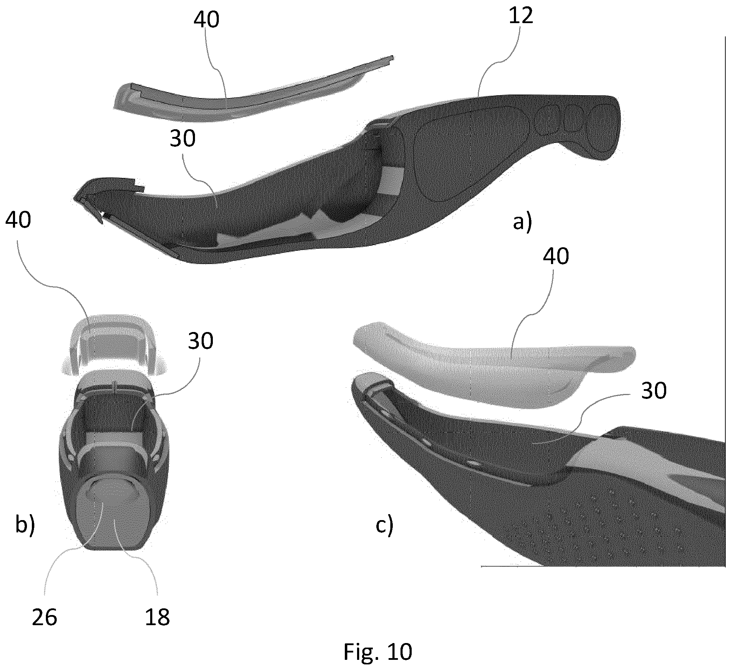

An die Hobelklinge 18 grenzt eine Spänekammer 30 an, sodass von der Schneide 26 der Hobelklinge 18 abgeschabte Knochenspäne in die Spänekammer 30 gelangen. Die Spänekammer 30 hat ein Volumen von etwa 4 cm³. Die Spänekammer 30 ist mit einem abnehmbaren Deckel 40 versehen; siehe Figuren 9 und 10.

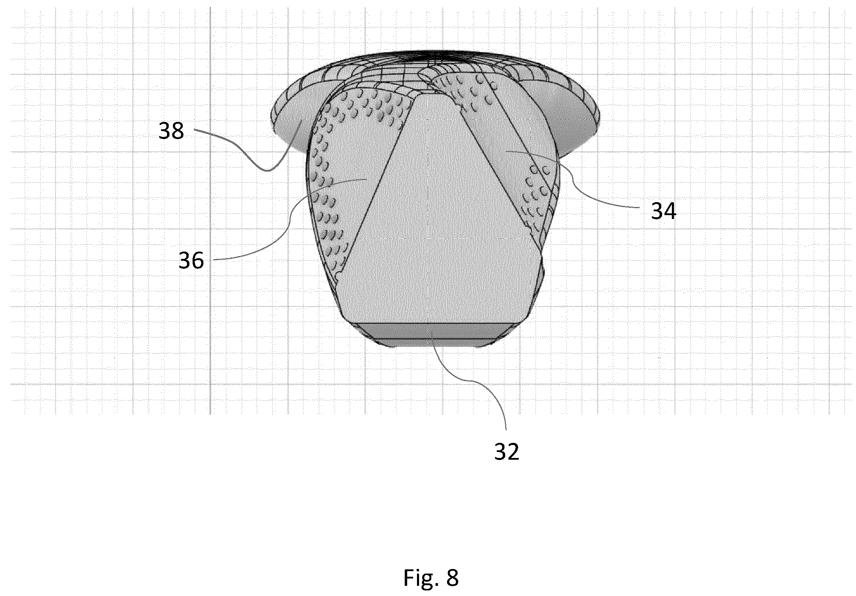

Das Handstück 20 weist drei als Mulden ausgebildete Fingerauflagen 32, 34 und 36 auf. Eine Fingerauflage 32 für den Mittelfinger eines Nutzers befindet sich auf der gleichen Seite des medizinischen Handinstruments 10, wie die Hobelklinge 18. Seitlich am Handstück 20 des medizinischen Handinstruments 10 sind eine Fingerauflage 34 für den Daumen des Nutzers und einer Fingerauflage 36 für den Zeigefinder des Nutzers angeordnet. Im Querschnitt des Handstücks 20 (sh. Figur 8) ist zu erkennen, dass die Fingerauflagen im Querschnitt so zueinander angeordnet sind, dass sie zwischen sich jeweils einen Winkel von kleiner als 90° einschließen.

In Längsrichtung 22 des Handstücks 20 sind die Fingerauflagen 32, 34 und 36 muldenförmig ausgebildet, d.h. konkav gewölbt. Dadurch bieten die Fingerauflagen 32, 34 und 36 jeweils eine gute Stütze für den jeweils anliegenden Finger des Nutzers. Am proximalen Ende 16 des Handinstruments 10 ist das Handstück 20 flach und relativ breit geformt und bildet so eine proximale Stützauflage 38 die beim Einsatz des medizinischen Handinstruments 10 den Fingerwurzelbereich zwischen Daumen und Zeigefinger des Nutzers auf dessen Hand aufliegen kann, sodass der Nutzer leicht die zum Hobeln nötige Andruckkraft aufbringen kann und sich die entsprechende Gegenkraft über die relativ große Fläche der Stützauflage auf die Hand des Nutzers übertragen wird.

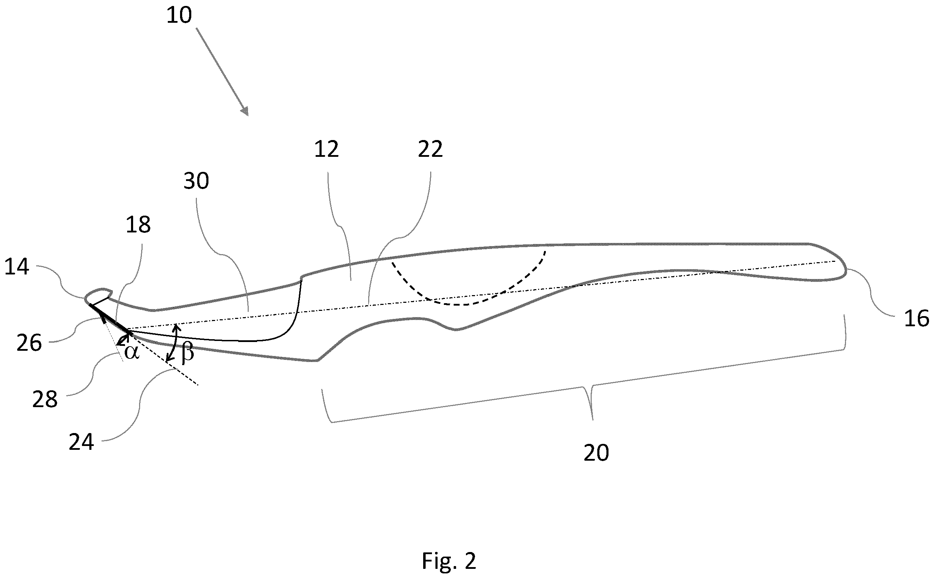

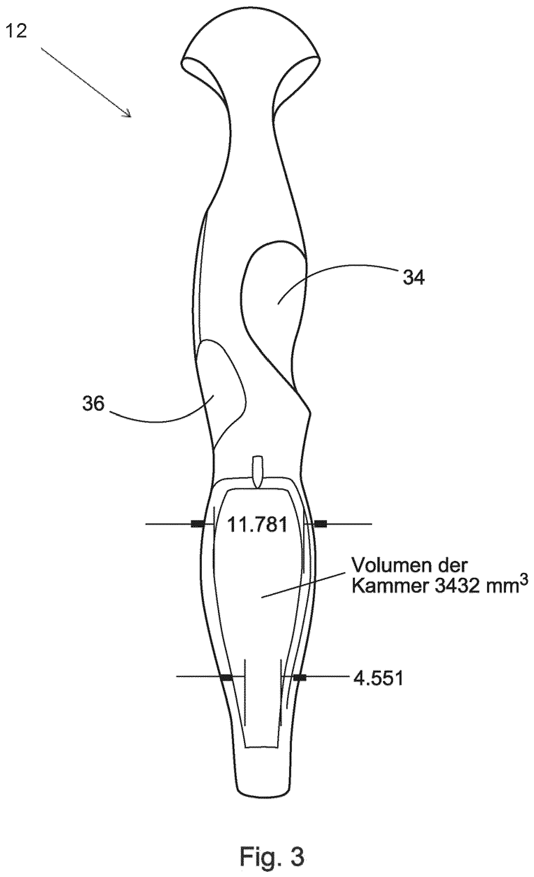

Figuren 1 und 2 zeigen den Grundkörper 12 mit daran befestigter Hobelklinge 18 einer ersten Ausführungsvariante des medizinischen Handinstruments 10. Der einstückige Grundkörper 12 dieser Ausführungsvariante ist in den Figuren 3 bis 7 noch einmal in verschiedenen Ansichten dargestellt. Der Grundkörper 12 ist vorzugsweise aus einem Kunststoff, insbesondere einem Polyamid oder einem Polycarbonat spritzgegossen.

In Figur 9 sind Ausführungsdetails der Spitze des Instruments 10 mit der Hobelklinge 18 und der Spänekammer 30 sowie dem Deckel 40 zu erkennen. Unmittelbar angrenzend an die Schneide 26 der Hobelklinge 18 befindet sich ein Spalt 42, durch den die von der Hobelklinge 18 abgeschabten Knochenspäne in die Spänekammer 30 gelangen können. Die Hobelklinge 18 kann wie in Figur 9 dargestellt in einem Winkel in beispielsweise Kunststoff eingebettet sein. Insbesondere kann eine Hobelklinge 18 aus rostfreiem Stahl von thermoplastischem Kunststoff umspritzt sein. Alternativ kann die Schneide 26 der Hobelklinge 18 auch aus einem größeren Stück Blech herausgebogen sein, wobei dann das Blech die Klingenebene 24 bildet (sh. Figur 2).

Figuren 10a bis 10c illustrieren in teilweise geschnittenen und perspektivischen Ansichten, wie die Spänekammer 30 mit dem Deckel verschlossen sein kann und wie die Hobelklinge 18 von einem Stück Blech gebildet sein kann, aus dem die eigentliche Schneide 26 herausgebogen ist, so ähnlich wie dies beispielsweise von Küchenreiben bekannt ist.

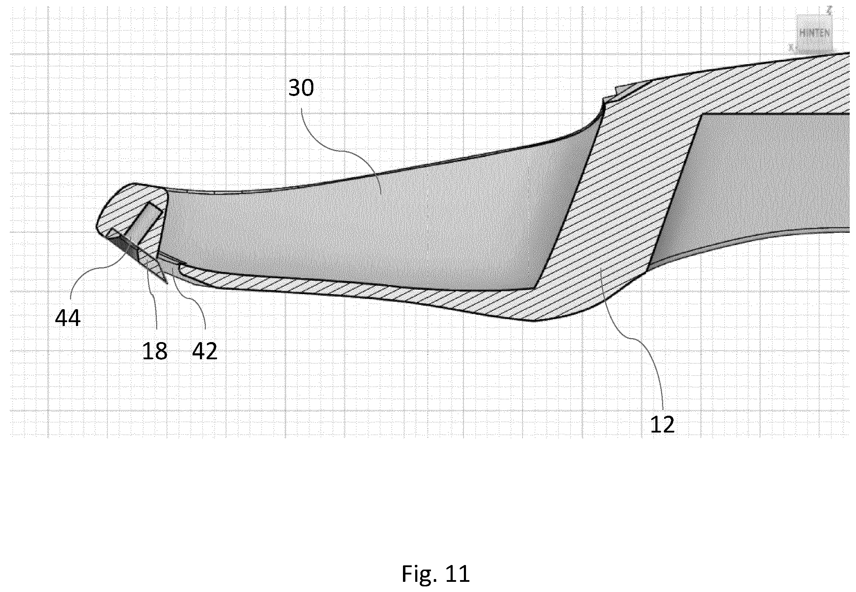

Figur 11 zeigt einen Längsschnitt durch einen distalen Endabschnitt des Grundkörpers 12 einer weiteren Ausführungsvariante des Handinstruments 10. In dem abgebildeten distalen Längsabschnitt befindet sich die Spänekammer 30. Außerdem ist nahe dem distalen Ende des Handinstruments 10 die Hobelklinge 18 mittels einer Schraube 44 an dem Grundkörper 12 befestigt. Zu erkennen ist auch der Spalt 42, über den von der Schneide 26 der Hobelklinge 18 abgetrennte Knochenspäne in die Spänekammer 30 gelangen können. Die in Figur 11 abgebildete Ausführungsvariante für die Befestigung der Hobelklinge 18 an dem Grundkörper 12 unterscheidet sich von der in Figur 9 abgebildeten Variante insbesondere dadurch, dass die Hobelklinge 18 mittels der Schraube 44 an dem Grundkörper 12 des Handinstruments 10 befestigt ist.

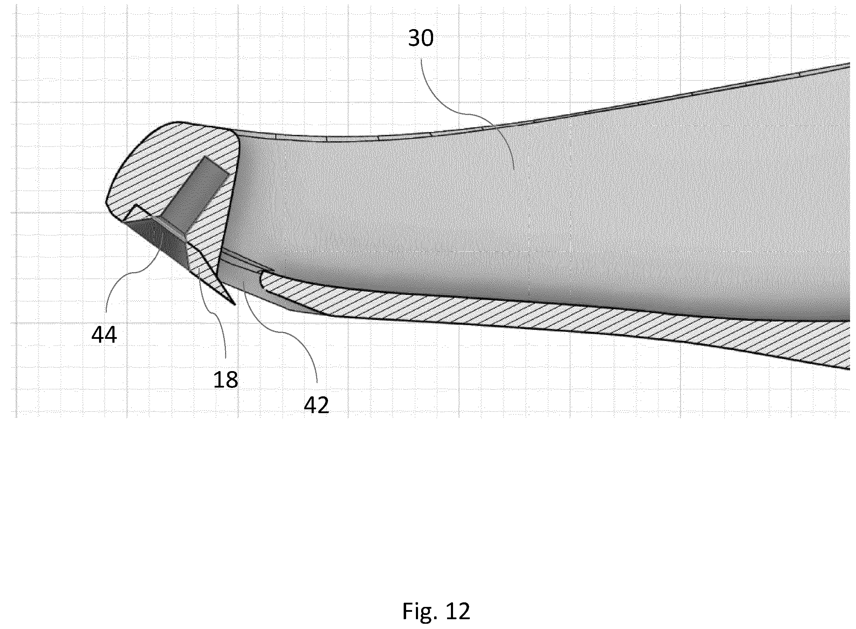

Dies ist noch genauer in der vergrößerten Abbildung in Figur 12 zu erkennen.

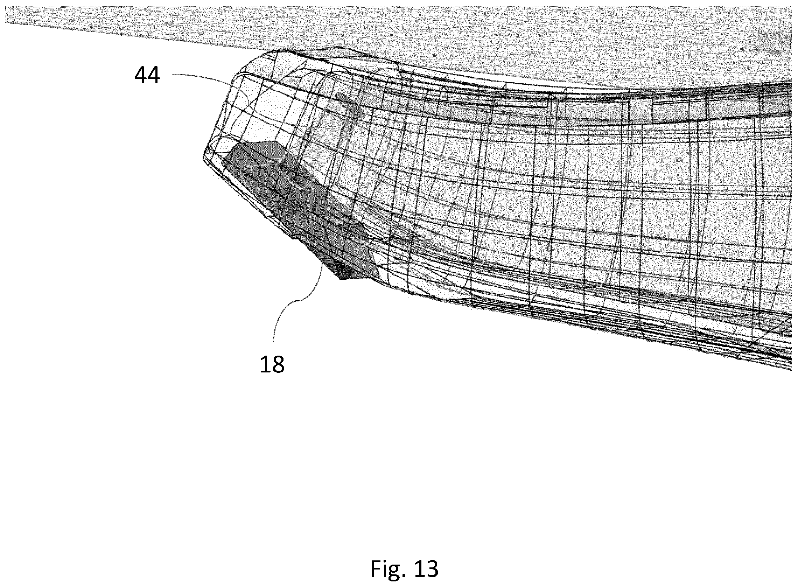

Figur 13 zeigt ein Gittermodell des Grundkörpers 12 des Handinstruments 10 und die Hobelklinge 18 sowie die Schraube 44 in einer perspektivischen Darstellung, die die räumliche Anordnung der Hobelklinge 18 und der Schraube 44 in Bezug auf das distale Ende des Grundkörpers 12 des Handinstruments 10 illustriert.

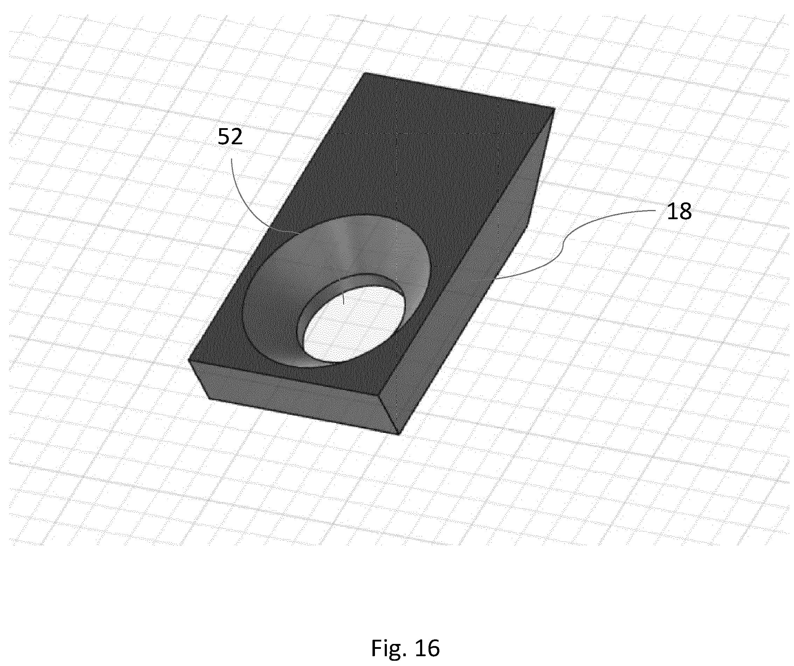

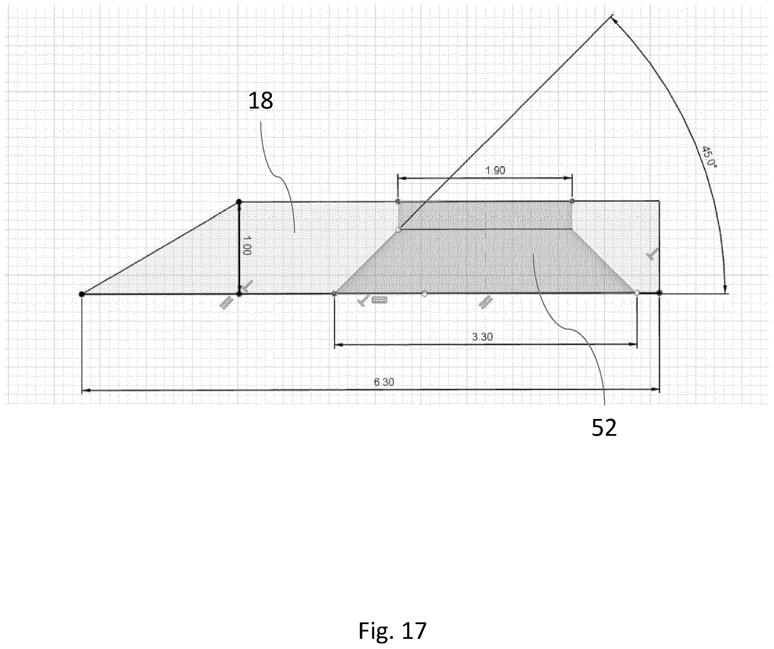

In Figur 16 ist die Hobelklinge 18 in einer perspektivischen Ansicht dargestellt und Figur 17 zeigt einen Längsschnitt durch die Hobelklinge 18.

Wie den Figuren 11 bis 13 zu entnehmen ist, ist es bei der dort dargestellten Ausführungsform vorgesehen, dass die Hobelklinge 18 mittels der Schraube 44 an dem Grundkörper 12 des Handinstruments 10 befestigt ist. Dadurch ist die Hobelklinge 18 grundsätzlich lösbar mit dem übrigen Handinstrument 10 verbunden. Wichtiger Aspekt ist jedoch, dass die Befestigung der Hobelklinge 18 mittels der Schraube 44 sicherstellt, dass die Hobelklinge 18 fest genug an dem Grundkörper 12 des Handinstruments 10 befestigt ist.

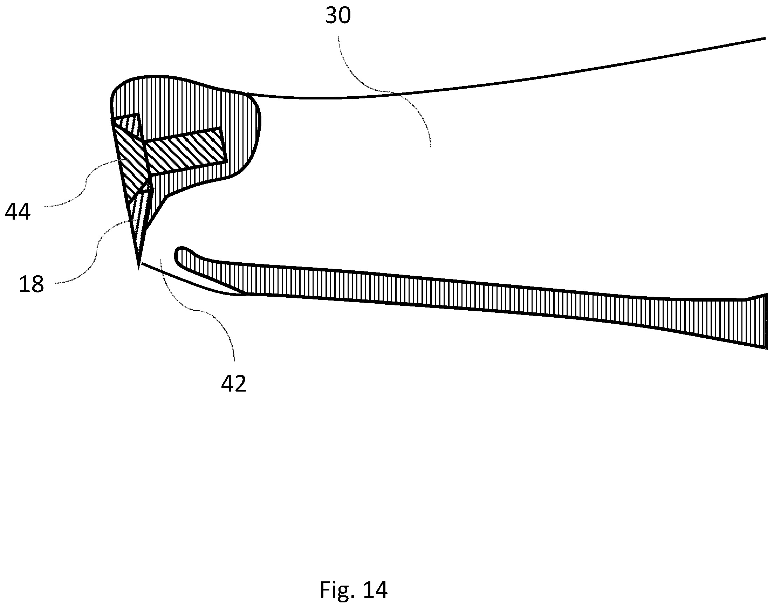

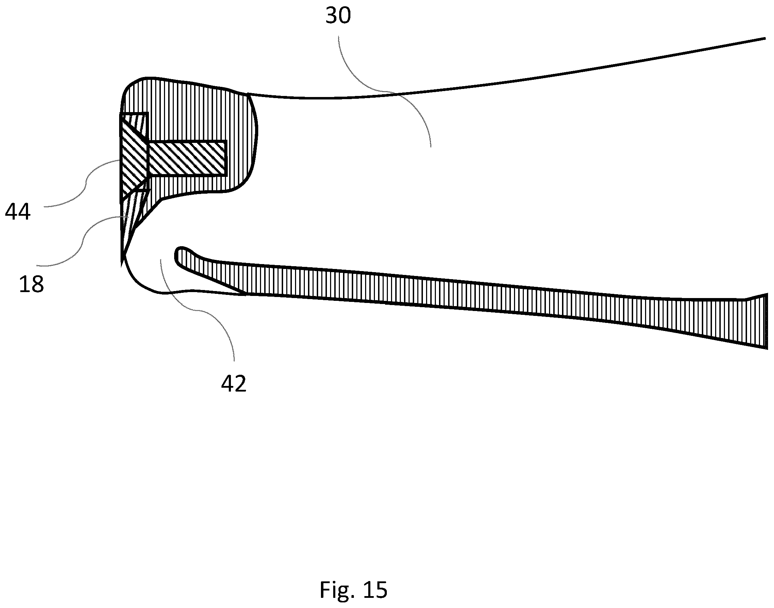

Verschiedene Ausführungsvarianten des Handinstruments 10 können sich dadurch unterscheiden, in welchem Winkel die Klingenebene 24 zur Längsachse 22 des Handstücks 20 verläuft. Figuren 14 und 15 illustrieren zwei Beispiele, in denen der Winkel zwischen der Klingenebene 24 und der Längsachse 22 des Handstücks 20 größer ist, als in den Beispielen, die in den Figuren 1 bis 13 abgebildet sind, nämlich bis zu 90° (siehe Figur 15).

Hierfür kommt es auch auf die Geometrie der Schraube 44 an, die in Figur 16 im Detail dargestellt ist. Wie zu erkennen ist, weist die Schraube 44 ein selbstschneidendes Gewinde 46 sowie einen Senkkopf 48 auf. In dem Senkkopf 48 ist eine Vertiefung 50 mit einem Mitnehmerprofil in Form eines Innensechsrunds vorgesehen, um die Schraube 44 mittels eines entsprechenden Werkzeugs der Größe T6 drehen zu können. Der Außendurchmesser des Senkkopfes 48 ist so bemessen, dass er sich fast über die gesamte Breite der Hobelklinge 18 erstreckt, um eine möglichst große Berührungsfläche zwischen den Schrägen des Senkkopfes 48, der Schraube 44 und den entsprechenden Schrägen der zugehörigen Befestigungsöffnung 52 in der Hobelklinge 18 zu gewährleisten. Die Befestigungsöffnung 52 ist dabei so gestaltet, dass der Senkkopf (Schraubenkopf) 48 der Schraube 44 nicht über die Kontur der Hobelklinge 18 hinausragt, wenn die Hobelklinge 18 mittels der Schraube 44 mit dem Grundkörper 12 des Handinstruments 10 verschraubt ist. Dies ist beispielsweise in Figur 12 dargestellt.

Die dargestellte Befestigung der Hobelklinge 18 mittels der Schraube 44 bietet zwei Vorteile. Auf Grund der vorteilhaften Gestaltung von Hobelklinge 18 und Schraube 44 ist die Befestigung der Hobelklinge 18 an dem Grundkörper 12 mittels der Schraube 44 besonders fest. Da die Schraube 44 ein selbstschneidendes Gewinde 46 aufweist, kann der Grundkörper 12 des Handinstruments 10 leicht aus einem thermoplastischen Kunststoff durch Spritzgießen hergestellt werden, ohne dass dabei auch ein Gewinde für die Schraube 44 erzeugt werden muss. Ein weiterer Vorteil ist, dass es theoretisch möglich ist, während der Behandlung eines Patienten eine möglicherweise stumpf gewordene Hobelklinge 18 durch eine neue Hobelklinge 18 zu ersetzen. Grundsätzlich ist jedoch das Handinstrument 10 für eine Einmalverwendung vorgesehen, bei der die Hobelklinge 18 dauerhaft mit dem Grundkörper 12 verbunden bleibt und nach Verwendung des Handinstruments an einem Patienten zusammen mit dem übrigen Handinstrument 10 entsorgt wird.

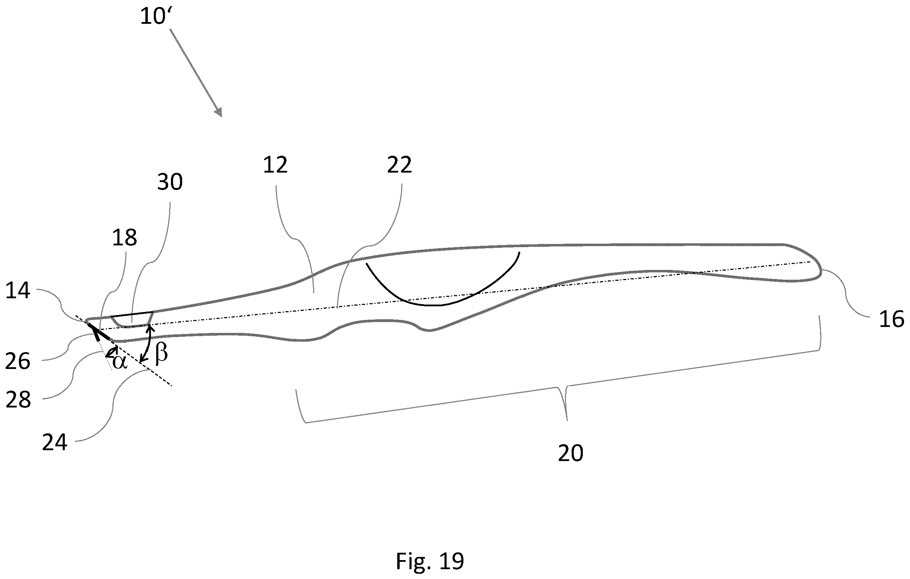

Je nach Patientin oder Patient kann ein medizinisches Handinstrument, wie es in den Abbildungen 1 bis 13 dargestellt ist, zu groß sein. Deshalb kann das medizinische Handinstrument 10' auch alternativ so gestaltet sein, dass sein gesamter distaler Bereich einschließlich der Hobelklinge 26' und der Spänekammer 30' wesentlich schmaler ausgeführt sind. Ein solches medizinisches Handinstrument 10' ist in Figur 19 abgebildet.

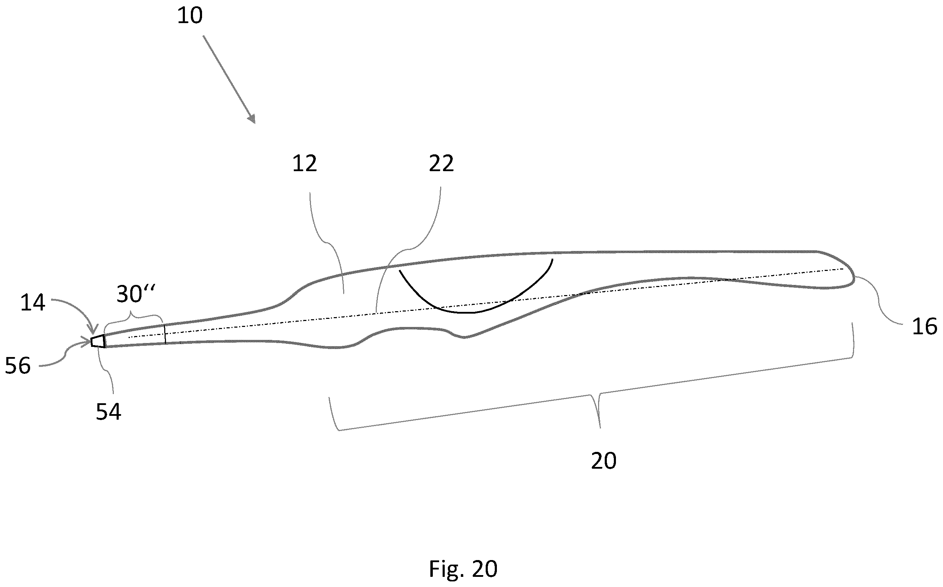

Gemäß einer Untervariante der ersten Variante des medizinischen Handinstruments 10" besitzt das medizinische Handinstrument 10‴ an seinem distalen Ende eine Ringklinge 54 die an einem distalen Ende einer hülsenförmigen Spänekammer 30" befestigt ist; siehe Figur 20. Die ringförmige Hobelklinge 54 umschließt eine zentrale Öffnung 56, durch die hindurch abgeschabte Knochenspäne in die Spänekammer 30" gelangen können.

Die hülsenförmige Spänekammer 30" ist samt der an ihr befestigten Ringklinge 54 von dem übrigen medizinischen Handinstrument 10" trennbar. Beispielsweise kann die hülsenförmige Spänekammer 30" auf das übrige Handinstrument aufgesteckt oder aufgeschraubt sein. Mit einem derartigen medizinischen Handinstrument 10" kann ein noch schmalerer distaler Bereich realisiert sein, um Knochen auch an ansonsten unzugänglichen Stellen abschaben zu können. Der Nachteil ist in diesem Falle ein geringes Fassungsvolumen der Spänekammer 30".

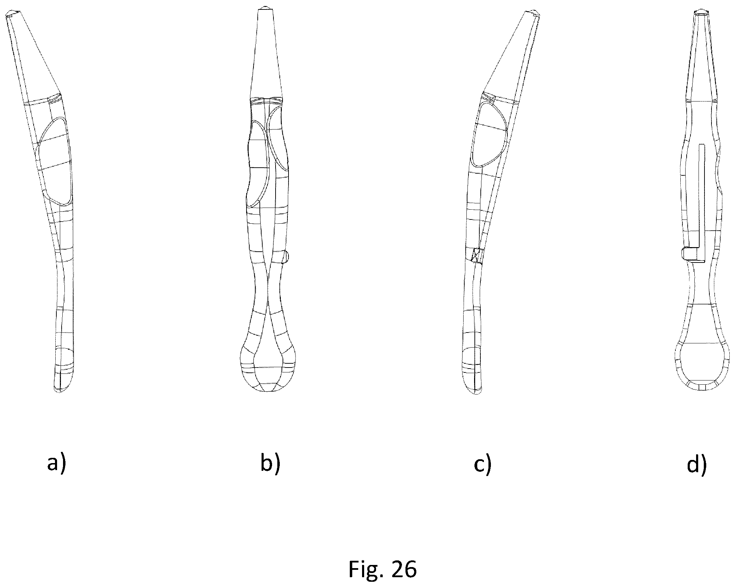

Nun zur zweiten Variante des medizinischen Handinstruments 10:
Auch bei der zweiten Variante des medizinischen Handinstruments 10 erstreckt sich das Handstück 20 entlang einer Längsachse 22 und ist ähnlich geformt, wie das Handstück der in den Figuren 1 bis dargestellten Variante. Dies ist in den perspektivischen Ansichten in den Figuren 21, 22, 23 und 28 sowie den Seitenansichten in den Figuren 26a bis 26d erkennbar. Ein Unterschied zu den zuvor in den Figuren 1 bis 20 dargestellten Varianten und Untervarianten des medizinischen Handinstruments 10 besteht bezüglich der Art und Anordnung der Hobelklinge und der Spänekammer.

Bei der zweiten Variante des medizinischen Handinstruments 10 ist die Hobelklinge 60 von einer konusförmigen Scheibe gebildet, deren Umfangskante die Schneidkante 62 bildet und die einen Durchmesser zwischen 4 mm und 6 mm, z.B. von 5 mm hat. In alternativen Ausführungsvarianten kann die Hobelklinge 60 auch einen größeren Durchmesser, z.B. 8 mm, 10 mm oder 12 mm haben. Die Hobelklinge 60 ist mit einem Schaft 64 verbunden, der mit dem Zentrum der Hobelklinge 60 auf der inneren Seite im Bereich der Spitze 66 der die Hobelklinge 60 bildenden konusförmigen Scheibe verbunden ist.

Die Spänekammer 30‴ hat die Form eines Hohlzylinders, in deren Zentrum der Schaft 64 verläuft. Der Durchmesser des Schafts 64 ist kleiner als der Innendurchmesser der Spänekammer 30', so dass sich zwischen der Mantelfläche des Schafts 64 und der Innenwand der Spänekammer 30‴ ein Hohlraum ergibt, der zum Sammeln von Spänen dient.

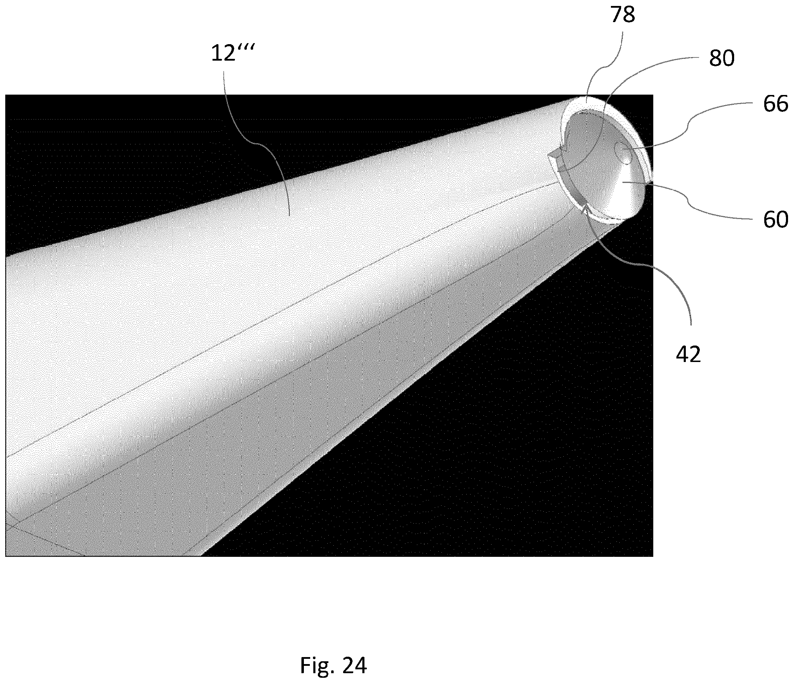

Das medizinische Handinstrument 10 ist im Bereich des distalen Endes der Spänekammer 30‴ so geformt, dass ein Vorsprung 78 am distalen Ende des Grundkörpers 12‴ die Hobelklinge 60 in einem ersten Teilumfang der Hobelklinge 60 umschließt, während ein zweiter Teilumfang der Hobelklinge 60 freisteht, wenn sich die Hobelklinge 60 in ihrer Arbeitsposition befindet; siehe Figur 24. Im Bereich des zweiten, freistehenden Teilumfangs der Hobelklinge 60 ist das distale Ende des Grundkörpers 12 derart verkürzt, dass sich ein Teil ringförmiger Spalt 42‴ ergibt, durch den hindurch Knochenspäne in die Spänekammer 30‴ gelangen können. Das distale Ende des Grundkörpers 12‴ des medizinischen Handinstruments 10 ist somit stufenförmig gestaltet, wobei der distal nach vorne abstehende Vorsprung 78 des Grundkörpers 12‴ den ersten Teilumfang der Hobelklinge 60 umschließt, während eine in Längsrichtung des Grundkörpers 12 zurückspringende Stirnwand 80 des Grundkörpers 12‴ den Spalt 42‴, der zur Spänekammer 30‴ führt, in Längsrichtung begrenzt. Der Spalt 42‴ befindet sich somit zwischen der Schneidkante 62 der Hobelklinge 60 und der in axialer Richtung zurückspringenden Stirnwand 80 des Grundkörpers 12‴ des Handinstruments 10.

Beim Schaben wirken in dem Schaft 64 Zugkräfte, die auf die Hobelklinge 60 übertragen werden. Von dem abzuschaben Material werden der Zugkraft entgegen gerichtete Kräfte auf die Hobelklinge 60 ausgeübt, die zu einem Drehmoment führen. Um dieses Drehmoment aufzufangen und die Hobelklinge 60 abzustützen, ist eine Stütz- und Anlagefläche 84 am distalen Ende des Grundkörpers 12‴ vorgesehen, die in einer senkrecht zur Längsachse des Schafts 64 verlaufenden Ebene liegt, die gegenüber der Ebene, in der die Stirnfläche 80 verläuft, um die Breite des sich dadurch ergebenden Spalts 42‴ distal versetzt ist. Die Stütz- und Anlagefläche 84 ist von dem nach vorne abstehende Vorsprung 78 des Grundkörpers 12‴ eingefasst. Der nach vorne abstehende Vorsprung 78 des Grundkörpers 12‴ fängt Kräfte auf, die quer zur Längsachse des Schafts 64 auf die Hobelklinge einwirken. Durch den Schaft 64, die und den nach vorne abstehende Vorsprung 78 des Grundkörpers 12‴ ist die Hobelklinge in ihrer Arbeitsposition für das Schaben optimal stabilisiert und so gehalten, dass die beim Schaben wirkenden Kräfte in den Grundkörper 12‴ eingeleitet werden, ohne dass sich Schaft 64 und Hobelklinge 60 unter dem Einfluss der einwirkenden Kräfte stärker verformen können.

Damit können von einem Benutzer über das ergonomisch geformte Handstück 20 in die Hobelklinge 60 eingeleitete Kräfte sehr kontrolliert zum zielgenauen und effektiven Schaben umgesetzt werden. Die ergonomische Form des Handstücks 20 und die Ausgestaltung der Hobelklinge 60 und des distalen Endes des Grundkörpers 12‴ haben somit einen synergistischen Effekt, der eine besonders präzise und effektive Handhabung des Instruments 10 erlaubt.

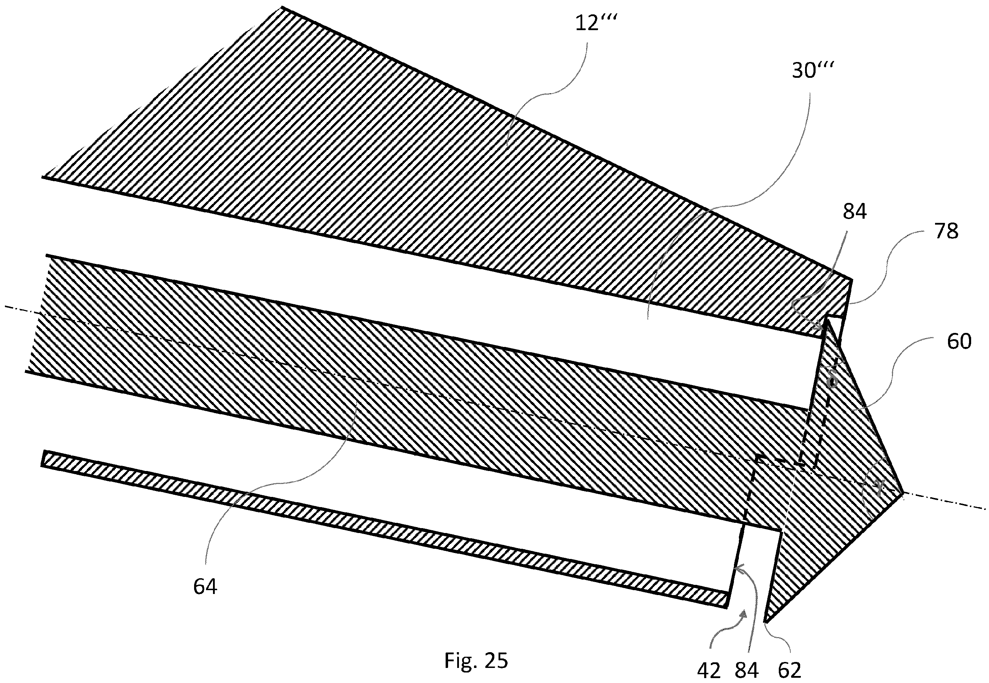

Die Hobelklinge 60 ist als konusförmige Scheibe ausgebildet und kann wie in Figur 25 abgebildet die Form eines stumpfen Kegels mit einem Spitzenwinkel γ von etwa 110° haben. Anders als in Figur 25 dargestellt ist die Spitze 66 der Hobelklinge 60 vorzugsweise abgerundet. Der Spitzenwinkel von 110° führt zu einem Schneidenwinkel δ dem umlaufenden Schneidkante 62 von 35°, sofern die Hobelklinge nicht hohl ausgeführt ist, sondern wie abgebildet eine plane Rückseite aufweist.

Der Außendurchmesser des Auswerfers 68 entspricht annähernd einen Innendurchmesser der Spänekammer 30"', so dass eine Stirnwand 70 des Auswerfers 68 die Spänekammer 30‴ in ihrer Längsrichtung begrenzt, wenn sich die Hobelklinge 60 in der Arbeitsposition befindet, der Auswerfer 68 aber in der Spänekammer 30‴ verschoben werden kann.

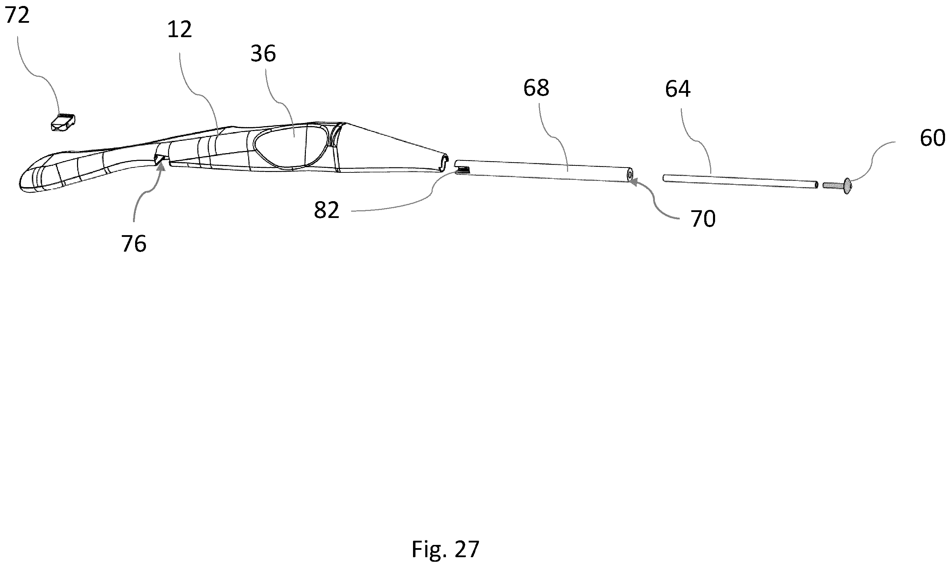

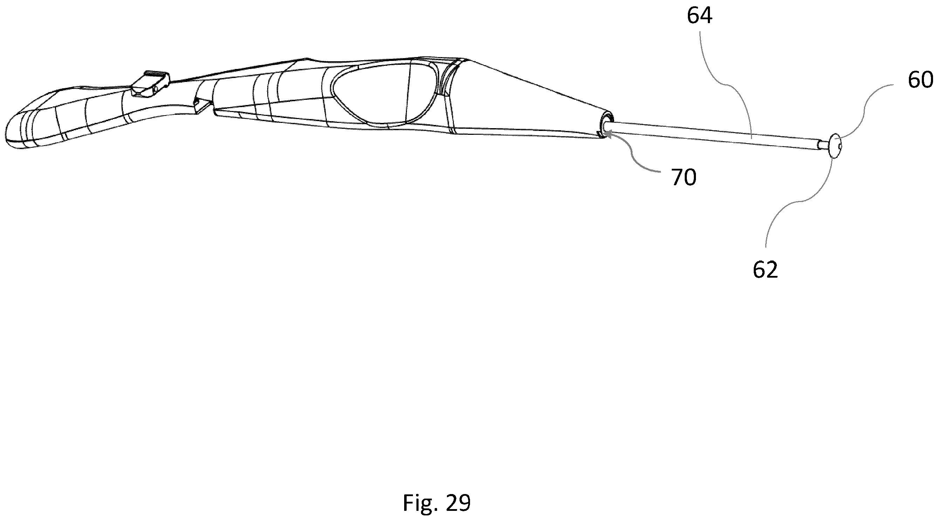

Zum Auswerfen von Knochenspänen können der Auswerfer 68 zusammen mit dem Schaft 64 in der Hobelklinge 60 axial nach vorne aus dem Grundkörper 12‴ herausgeschoben werden, so wie dies in Figur 29 dargestellt ist. Hierzu ist ein Griff 72 vorgesehen, der am proximalen Endes des Auswerfers 68 befestigt und in einer Längsnut 74 in dem Grundkörper 12‴ des Handinstruments 10 geführt ist. Mit Hilfe des Griffs 72 können die Hobelklinge 60, der Schaft 64 und der Auswerfer 68 axial aus einer Arbeitsposition in die Auswerfposition und zurück verschoben werden. Dabei bewegt sich der Griff 72 in der Längsnut 74. Der Griff 72 kann ein integraler Bestandteil des Auswerfers 68 sein oder er kann als separates Teil an dem Auswerfer 68 befestigt sein. Hierzu kann der Auswerfer 68 an seinem proximalen Ende eine Ausnehmung 82 aufweisen, in die ein radial nach innen weisendes Ende des Griffs 72 eingesetzt werden kann, siehe Figur 27.

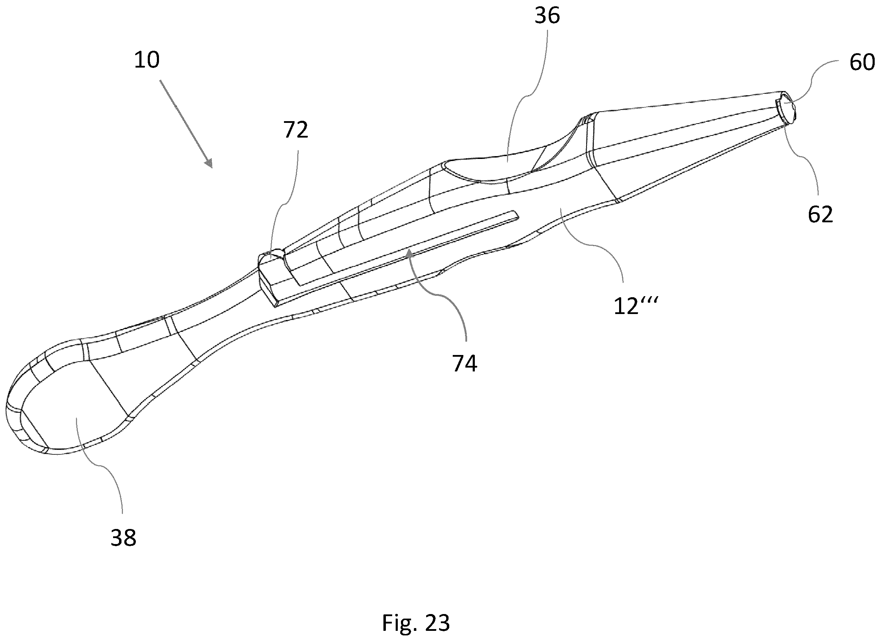

Um die Hobelklinge 60 mit dem Schaft 64 in dem Auswerfer 68 in der Arbeitsposition der Hobelklinge 60 zu fixieren, kann der Griff 72 dann, wenn sich die Hobelklinge 60 in der Arbeitsposition befindet, um die Längsachse des Schafts 64 und des Auswerfers 68 herum in eine arretierende Ausnehmung 76 hineingeschwenkt werden; siehe Figur 23. Wenn der Griff 72 in die Ausnehmung 76 geschwenkt ist, kann der Griff 72 nicht mehr entlang der Längsnut 74 hin und her bewegt werden.

Um das medizinische Handinstrument 10 gemäß der zweiten Variante zum Schaben zu verwenden, muss sich die Hobelklinge 60 zusammen mit dem Schaft 64 und dem Auswerfer 68 in der Arbeitsposition befinden, in der die Hobelklinge teilweise vom distalen Ende des Grundkörpers 12‴ des Handinstruments 10 umschlossen ist. Der Griff 72 muss in die arretierende Ausnehmung 76 geschwenkt sein, damit Zugkräfte vom Grundkörper 12‴ des Handinstruments 10 über eine distale Wand der arretierenden Ausnehmung 76 auf den Griff 72 und von diesem über den Auswerfer 68 und den Schaft 64 auf die Hobelklinge 60 übertragen werden kann. Dieser Zustand ist in Figuren 21 bis 25 und 27 dargestellt.

Um nach Gebrauch des medizinischen Handinstruments 10 dessen Spänekammer 30‴ entleeren zu können, muss der Griff 72 aus der arretierenden Ausnehmung 76 herausgeschwenkt und so positioniert werden, dass der Griff 72 in der Längsnut 74 nach vorne geschoben werden kann. Dabei werden auch der Auswerfer 68, der Schaft 64 und die Hobelklinge 60 nach vorne - also in distale Richtung - bewegt. Dabei schiebt die Stirnwand 70 des Auswerfers 68 in der Spänekammer 30‴ befindliches Material in distale Richtung aus der Spänekammer 30‴ heraus.

In dem abgebildeten Ausführungsbeispiel ist der Auswerfer 68 als ein länglicher zylindrischer Körper ausgebildet, dessen Außendurchmesser in etwa dem Innendurchmesser der Spänekammer 30‴ entspricht und an dem distal der Schaft 64 befestigt ist. Diese Ausbildung des Auswerfers 68 hat den Vorteil, dass die Umfangsfläche des Auswerfers 68 und die Innenwand der Spänekammer 30‴ eine sichere Längsführung gewähren. In der Explosionszeichnung in Figur 27 sind alle Komponenten dieser Ausführungsform des medizinischen Handinstruments gezeigt, u.a. auch der längliche Auswerfer 68 mit seiner Stirnfläche 70.

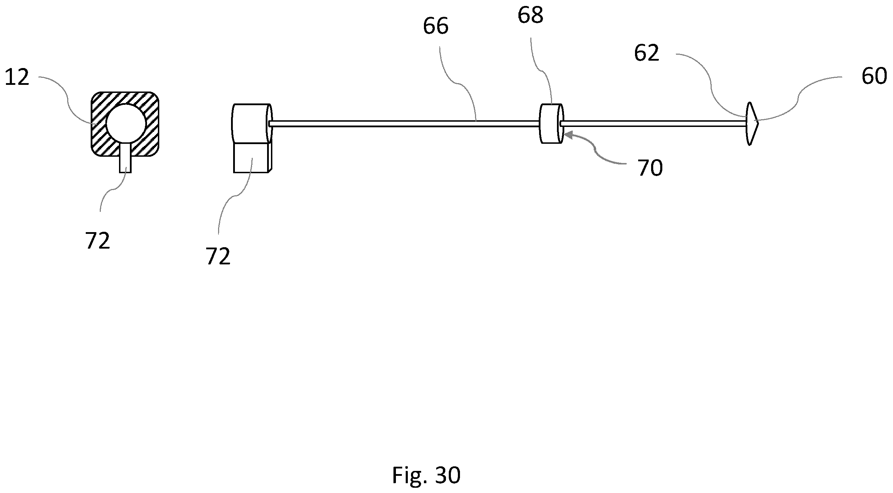

In einer alternativen, in Figur 30 dargestellten Ausführungsform, kann der Auswerfer 68 auch als eine Art Scheibe ausgebildet sein, die an dem Schaft 64 befestigt ist. Bei dieser Ausführungsform erstreckt sich der Schaft 64 durch den scheibenförmigen Auswerfer 68 hindurch bis hin zu dem Griff 72. Hierbei kann eine ausreichende Längsführung dadurch gewährt werden, dass der Griff 72 so ausgebildet ist, dass er entlang der Längsnut 64 nicht nur seitlich, sondern in alle radialen Richtungen sicher geführt ist. Eine entsprechende Gestaltung des Grundkörpers 12 und des Griffs 72 sind in Figur 30a in einem Querschnitt skizzenhaft dargestellt.

In einer weiteren, nicht dargestellten Ausführungsvariante ist der Schaft durchgehend ausgeführt und an seinem proximalen Ende abgewinkelt, so dass das abgewinkelte Ende des Schafts den Griff zum Vorschieben und Zurückziehen von Hobelklinge und Auswerfer bildet. Weil der Schaft in dieser Ausführungsform von der Hobelklinge bis zu seinem den Griff bildenden abgebwinkelten Ende durchgehend und einstückig ausgebildet ist, kann er große Zugkräfte übertragen.

Die Hobelklinge 60 ist vorzugsweise aus rostfreiem medizinischem Stahl, beispielsweise aus 316L hergestellt. Auch der Schaft 64 besteht vorzugsweise aus rostfreiem Stahl. Der Auswerfer 68 und der Griff 72 bestehen vorzugsweise aus Kunststoff. In einer Ausführungsvariante können der Auswerfer 68 und der Griff 72 einstückig ausgebildet sein, d.h. der Griff 72 ist als seitlicher Vorsprung am distalen Ende des Auswerfers 68 ausgebildet. In der anderen Ausführungsvariante, insbesondere der Ausführungsvariante, bei der der Auswerfer die Form einer Scheibe oder eines kurzen Zylinders hat, die bzw. der an dem Schaft 64 befestigt ist, sind der Auswerfer 68 und der Griff 72 separat mit dem Schaft 64 verbunden.

### Bezugszeichen

- 10: Handinstrument
- 12: Grundkörper
- 14: distales Ende
- 16: proximales Ende
- 18: Hobelklinge
- 20: Handstück
- 22: Längsachse
- 24: Klingenebene
- 26: Schneide
- 28: Schnittwinkel
- 30: Spänekammer
- 32: Fingerauflage für Mittelfinger
- 34: Fingerauflage für Daumen oder Zeigefinger
- 36: Fingerauflage für Daumen oder Zeigefinger
- 38: Stützauflage
- 40: Deckel der Spänekammer
- 42: Spalt
- 44: Schraube
- 46: Gewinde
- 48: Senkkopf
- 50: Vertiefung
- 52: Befestigungsöffnung
- 54: Ringklinge
- 56: zentrale Öffnung in der Ringklinge
- 60: scheibenförmige Hobelklinge (konusförmige Scheibe)
- 62: Schneidkante der Hobelklinge
- 64: Schaft, an dessen distalem Ende die Hobelklinge befestigt ist
- 66: Spitze der die Hobelklinge bildenden konusförmigen Scheibe
- 68: Auswerfer zum Auswerfen von Spänen aus der Spänekammer
- 70: Stirnwand des Auswerfers
- 72: Griff zum Vor- und Zurückschieben von Schaft, Auswerfer und Hobelklinge
- 74: Längsnut für den Griff
- 76: Ausnehmung zum Arretieren des Griffs
- 78: Vorsprung an distalen Ende des Grundkörpers
- 80: Stirnwand des Grundkörpers
- 82: Ausnehmung am proximalen Ende des Auswerfers zur Befestigung des Griffs
- 84: Stütz- und Anlagefläche für die Hobelklinge
- α: Schnittwinkel
- β: Führungswinkel
- γ: Spitzenwinkel
- δ: Schneidenwinkel

## Patentansprüche

1. Handinstrument (10) mit einem Handstück (20) und einer distal des Handstücks angeordneten Hobelklinge (18; 60) und einer Spänekammer (30), von denen die Hobelklinge (18; 60) zum Schaben von Knochen ausgebildet und so angeordnet ist, dass mittels der Hobelklinge geschabte Späne in die Spänekammer (30) gelangen und die Hobelklinge (18; 60) in einer gegenüber einer Längsachse (22) des Handstücks (20) geneigten Ebene verläuft,
**dadurch gekennzeichnet, dass** das Handstück (20) als Griff mit drei Fingerauflagen (32, 34, 36) und einer proximalen Stützauflage (38) ausgebildet ist, wobei die drei Fingerauflagen (32, 34, 36) so zueinander geneigt sind, dass die drei Fingerauflagen (32, 34, 36) in einem Querschnitt durch das Handstück (20) paarweise in einem Winkel von kleiner als 90° zueinander verlaufen.

2. Handinstrument nach Anspruch 1, **dadurch gekennzeichnet, dass** die drei Fingerauflagen (32, 34, 36) als Mulden ausgebildet sind in Längsrichtung des Handstücks (20) eine konkave Form haben.

3. Handinstrument nach Anspruch 1 oder 2, **dadurch gekennzeichnet, dass** die Stützauflage (38) von einer balligen Fläche am proximalen Ende des Handstücks (20) gebildet ist, die in diejenige radiale Richtung des Handstücks (20) orientiert ist, in die eine äußere Flachseite der Hobelklinge (18) orientiert ist.

4. Handinstrument nach Anspruch 3, **dadurch gekennzeichnet, dass** die Stützauflage (38) in Längsrichtung des Handinstruments (10) in einem Winkel stumpfen Winkel zwischen 0° und 10° in Bezug auf die äußere Flachseite der Hobelklinge (18) verläuft.

5. Handinstrument nach wenigstens einem der Ansprüche 1 bis 4, **dadurch gekennzeichnet, dass** die Spänekammer (30) ein Volumen von mehr als 3,5 cm³, vorzugsweise mehr als 4 cm³ aufweist.

6. Handinstrument nach wenigstens einem der Ansprüche 1 bis 5, **dadurch gekennzeichnet, dass** die Hobelklinge (18) in Wesentlichem, insbesondere mit Ausnahme der Schneide (26), in einer Klingenebene (24) verläuft, die in Bezug auf die Längsachse (22) des Handstücks um mindestens 50° geneigt ist.

7. Handinstrument nach wenigstens einem der Ansprüche 1 bis 6, **dadurch gekennzeichnet, dass** das Handstück (20) aus thermoplastischem Kunststoff besteht, und zwar vorzugsweise aus in der Medizin zugelassenen Polycarbonat Makrolon 2458 oder dem Polyamid ixef 1022.

8. Handinstrument nach wenigstens einem der Ansprüche 1 bis 7, **dadurch gekennzeichnet, dass** die Spänekammer (30) und das Handstück (20) einstückig ausgebildet sind und Bestandteil eines einstückigen Grundkörpers (12) sind, an dem die Hobelklinge (18) und ein Spänekammerverschluss in Form eines abnehmbaren Deckels (40) befestigt sind.

9. Handinstrument nach wenigstens einem der Ansprüche 1, 2 und 5 bis 8, **dadurch gekennzeichnet, dass** die Hobelklinge (60) als konusförmige Scheibe ausgebildet und an einem Schaft (64) befestigt ist, der in dem Handstück (20) längsverschieblich angeordnet ist.

10. Handinstrument nach Anspruch 9, **dadurch gekennzeichnet, dass** die Spänekammer (30‴) als ein zylindrischer, den Schaft (64) konzentrisch umgebender Hohlraum ausgebildet ist, der am distalen Ende des Handstücks (20) offen ist.

11. Handinstrument nach Anspruch 9 oder 10, **gekennzeichnet durch** einen Auswerfer (68), der zusammen mit der Hobelklinge (60) und dem Schaft (64) längsverschieblich ist und einen axialen Abstand von der Hobelklinge (60) hat, wobei sich die Spänekammer in axialer Richtung zwischen der Hobelklinge (60) und einer Stirnwand (70) des Auswerfers (68) erstreckt.

12. Handinstrument nach wenigstens einem der Ansprüche 9 bis 11, **dadurch gekennzeichnet, dass** der Schaft (64) mit der Hobelklinge (60) derart arretierbar ist, dass der Schaft (64) und die Hobelklinge (60) im arretierten Zustand nicht mehr bezüglich des Handstücks (20) längsverschieblich sind und die Hobelklinge in einer zum Schaben dienenden Arbeitsposition derart arretiert ist, dass mittels des Handstücks (20) Zugkräfte in den Schaft (64) eingeleitet und auf die Hobelklinge (60) übertragen werden können.

13. Handinstrument nach wenigstens einem der Ansprüche 9 bis 11, **dadurch gekennzeichnet, dass** der Schaft (64) wenigstens mittelbar mit einem Griff (72) verbunden ist, der zum Längsverschieben des Schafts (64) und der Hobelklinge (60) bezüglich des Handstücks (20) dient.

14. Handinstrument nach Anspruch 13, **dadurch gekennzeichnet, dass** das Handstück (20) eine Längsnut (74) zur Führung des Griffs (72) aufweist.

15. Handinstrument nach Anspruch 14, **dadurch gekennzeichnet, dass** der Griff (72) das Handstück (20) eine Ausnehmung am proximalen Ende der Längsnut aufweist, in die der Griff (72) mittels einer Drehbewegung um die Längsachse des Schafts (64) herum hineingeschwenkt werden kann, um den Schaft (64) und die Hobelklinge (60) in der Arbeitsposition zu arretieren.

## Claims

1. Hand instrument (10) having a handpiece (20) and a scraping blade (18; 60) arranged distally of the handpiece and a chip chamber (30), of which the scraping blade (18; 60) is designed for scraping bones and is arranged in such a way that chips scraped by means of the scraping blade enter the chip chamber (30) and that the scraping blade (18; 60) extends in a plane inclined with respect to a longitudinal axis (22) of the handpiece (20),
**characterized in that** the handpiece (20) is designed as a handle with three finger rests (32, 34, 36) and a proximal support rest (38), the three finger rests (32, 34, 36) being inclined to one another in such a way that the three finger rests (32, 34, 36) extend in pairs at an angle of less than 90° to one another in a cross-section through the handpiece (20).

2. Hand instrument according to claim 1, **characterized in that** the three finger rests (32, 34, 36) are designed as depressions having a concave shape in the longitudinal direction of the handpiece (20).

3. Hand instrument according to claim 1 or 2, **characterized in that** the support rest (38) is formed by a spherical surface at the proximal end of the handpiece (20), which is oriented in the radial direction of the handpiece (20) in which an outer flat side of the scraping blade (18) is oriented.

4. Hand instrument according to claim 3, **characterized in that** the support rest (38) extends in the longitudinal direction of the hand instrument (10) at an obtuse angle of between 0° and 10° with respect to the outer flat side of the scraping blade (18).

5. Hand instrument according to at least one of claims 1 to 4, **characterized in that** the chip chamber (30) has a volume of more than 3.5 cm³, preferably more than 4 cm.³

6. Hand instrument according to at least one of claims 1 to 5, **characterized in that** the scraping blade (18) extends substantially, in particular with the exception of the cutting edge (26), in a blade plane (24) which is inclined by at least 50° with respect to the longitudinal axis (22) of the handpiece.

7. Hand instrument according to at least one of claims 1 to 6, **characterized in that** the handpiece (20) is made of thermoplastic material, preferably of polycarbonate Makrolon 2458 or polyamide ixef 1022 approved for medical use.

8. Hand instrument according to at least one of claims 1 to 7, **characterized in that** the chip chamber (30) and the handpiece (20) are formed in one piece and are part of a one-piece base body (12) to which the scraping blade (18) and a chip chamber closure in the form of a removable cover (40) are attached.

9. Hand instrument according to at least one of claims 1, 2 and 5 to 8, **characterized in that** the scraping blade (60) is designed as a conical disc and is attached to a shaft (64) which is arranged in the handpiece (20) so as to be longitudinally displaceable.

10. Hand instrument according to claim 9, **characterized in that** the chip chamber (30"') is formed as a cylindrical cavity concentrically surrounding the shaft (64), which is open at the distal end of the handpiece (20).

11. Hand instrument according to claim 9 or 10, **characterized by** an ejector (68), which is longitudinally displaceable together with the scraping blade (60) and the shaft (64) and has an axial distance from the scraping blade (60), the chip chamber extending in the axial direction between the scraping blade (60) and an end wall (70) of the ejector (68).

12. Hand instrument according to at least one of claims 9 to 11, **characterized in that** the shaft (64) can be locked with the scraping blade (60) in such a way that the shaft (64) and the scraping blade (60) can no longer be longitudinally displaced with respect to the handpiece (20) in the locked state and the scraping blade is locked in a working position serving for scraping in such a way that tensile forces can be introduced into the shaft (64) by means of the handpiece (20) and transmitted to the scraping blade (60).

13. The hand instrument according to at least one of claims 9 to 11, **characterized in that** the shaft (64) is at least indirectly connected to a handle (72) which serves for longitudinally displacing the shaft (64) and the scraping blade (60) with respect to the handpiece (20).

14. Hand instrument according to claim 13, **characterized in that** the handpiece (20) has a longitudinal groove (74) for guiding the handle (72).

15. The hand instrument according to claim 14, **characterized in that** the handle (72) of the handpiece (20) has a recess at the proximal end of the longitudinal groove into which the handle (72) can be pivoted by means of a rotary movement around the longitudinal axis of the shaft (64) in order to lock the shaft (64) and the scraping blade (60) in the working position.

## Revendications

1. Instrument (10) à main comportant une pièce (20) à main et une lame (18 ; 60) de rabot disposée à l'extrémité distale de la pièce à main et une chambre (30) pour les copeaux, la lame (18 ; 60) de rabot étant conçue pour gratter de l'os et étant disposée de façon à ce que des copeaux grattés au moyen de la lame de rabot arrivent dans la chambre (30) pour les copeaux et que la lame (18 ; 60) de rabot s'étende dans un plan incliné par rapport à un axe (22) longitudinal de la pièce (20) à main,
**caractérisé en ce que** la pièce (20) à main est réalisée en tant que poignée comportant trois surfaces (32, 34, 36) pour les doigts et une surface (38) d'appui proximale, les trois surfaces (32, 34, 36) pour les doigts étant inclinées les unes par rapport aux autres, de telle manière que les trois surfaces (32, 34, 36) pour les doigts s'étendent dans une section transversale dans la pièce (20) à main par paires les unes par rapport aux autres suivant un angle inférieur à 90°.

2. Instrument à main suivant la revendication 1, **caractérisé en ce que** les trois surfaces (32, 34, 36) pour les doigts sont réalisées en auge et ont une forme concave dans la direction longitudinale de la pièce (20) à main.

3. Instrument à main suivant la revendication 1 ou 2, **caractérisé en ce que** la surface (38) d'appui est formée d'une face bombée à l'extrémité proximale de la pièce (20) à main, qui est orientée dans la direction radiale de la pièce (20) à main, dans laquelle une face plane extérieure de la lame (18) de rabot est orientée.

4. Instrument à main suivant la revendication 3, **caractérisé en ce que** la surface (38) d'appui s'étend dans la direction longitudinale de l'instrument (10) à main suivant un angle obtus entre 0° et 10° par rapport à la face plane extérieure de la lame (18) de rabot.

5. Instrument à main suivant au moins l'une des revendications 1 à 4, **caractérisé en ce que** la chambre (30) pour les copeaux a un volume de plus de 3,5 cm³, de préférence de plus de 4 cm³.

6. Instrument à main suivant au moins l'une des revendications 1 à 5, **caractérisé en ce que** la lame (18) de rabot s'étend essentiellement, notamment à l'exception de l'arête (26), dans un plan (24) de la lame, qui est incliné d'au moins 50° par rapport à l'axe (22) longitudinal de la pièce à main.

7. Instrument à main suivant au moins l'une des revendications 1 à 6, **caractérisé en ce que** la pièce (20) à main est en matière thermoplastique et cela, de préférence en polycarbonate 2458 autorisé en médecine ou en polyamide ixef 1022.

8. Instrument à main suivant au moins l'une des revendications 1 à 7, **caractérisé en ce que** la chambre (30) pour les copeaux et la pièce (20) à main sont réalisées d'un seul tenant et font partie d'un corps (12) de base d'un seul tenant, auquel sont fixées la lame (18) de rabot et une fermeture de la chambre pour les copeaux sous la forme d'un couvercle (40) amovible.

9. Instrument à main suivant au moins l'une des revendications 1, 2 et 5 à 8, **caractérisé en ce que** la lame (60) de rabot est réalisée en disque conique et est fixée à une tige (64), qui est montée coulissante longitudinalement dans la pièce (20) à main.

10. Instrument à main suivant la revendication 9, **caractérisé en ce que** la chambre (30‴) pour les copeaux est réalisée en cavité cylindrique, qui entoure concentriquement la tige (64) et qui est ouverte à l'extrémité distale de la pièce (20) à main.

11. Instrument à main suivant la revendication 9 ou 10, **caractérisé par** un éjecteur (68), qui peut coulisser longitudinalement conjointement avec la lame (60) de rabot et la tige (64) et qui a une distance axiale par rapport à la lame (60) de rabot, la chambre pour les copeaux s'étendant en direction axiale entre la lame (60) de rabot et une paroi frontale (70) de l'éjecteur (68).

12. Instrument à main suivant au moins l'une des revendications 9 à 11, **caractérisé en ce que** la tige (64) peut être bloquée de telle manière avec la lame (60) de rabot que la tige (64) et la lame (60) de rabot ne peuvent plus coulisser longitudinalement par rapport à la pièce (20) à main et que la lame de rabot peut être bloquée dans une position de travail servant au grattage de telle manière que, au moyen de la pièce (20) à main, des forces de traction peuvent être appliquées à la tige (64) et transmises à la lame (60) de rabot.

13. Instrument à main suivant au moins l'une des revendications 9 à 11, **caractérisé en ce que** la tige (64) est reliée au moins indirectement à une poignée (72), qui sert à faire coulisser longitudinalement la tige (64) et la lame (60) de rabot par rapport à la pièce (20) à main.

14. Instrument à main suivant la revendication 13, **caractérisé en ce que** la pièce (20) à main comporte une rainure (74) longitudinale pour guider la poignée (72).

15. Instrument à main suivant la revendication 14, **caractérisé en ce que** la poignée (72) de la pièce (20) à main comporte un évidement à l'extrémité proximale de la rainure longitudinale, dans lequel la poignée (72) peut être basculée au moyen d'un mouvement de rotation autour de l'axe longitudinal de la tige (64), pour bloquer la tige (64) et la lame (60) de rabot dans la position de travail.
